# EUROPEAN PATENT APPLICATION

(11) **EP 1 707 622 A1**
(43) Date of publication of application: **04.10.2006**
(21) Application number: 05701674.3
(22) Date of filing: 17.01.2005
(51) Int. Cl.: C12N 9/16, C12N 15/11, C12N 15/12, A61K 48/00, A61K 31/16, A61K 31/522, A61P 37/00

(54) **USE OF COMPOUNDS THAT REGULATE HDAC6 TUBULIN DEACETYLASE ACTIVITY IN THE PREPARATION OF PHARMACEUTICAL COMPOSITIONS, METHOD OF IDENTIFYING THE AFOREMENTIONED REGULATING COMPOUNDS, SAID PHARMACEUTICAL COMPOUNDS AND APPLICATIONS THEREOF**

(30) Priority: 20.01.2004 ES 200400107
(71) Applicant: UNIVERSIDAD AUTONOMA DE MADRID CIUDAD UNIVERSITARIA CANTOBLANCO, E-28049 Madrid (ES)
(72) Inventor: SERADOR PEIRÓ, Juan Manuel, E-46011 Valencia (ES); CABRERO GARCÍA, J.Román, E-28028 Madrid (ES); SANCHO MADRID, David, E-28028 Madrid (ES); MITTLEBRUNN HERRERO, María, E-28001 Madrid (ES); URZAINQUI MAYAYO, Ana, E-28034 Madrid (ES); SÁNCHEZ MADRID, Francisco, E-28035 Madrid (ES)
(74) Representative: Carvajal y Urquijo, Isabel
(86) International application number: PCT/ES2005/000019
(87) International publication number: WO 2005/078081

(57) **Abstract**

The object of the present invention is the use of an HDAC6 protein tubulin deacetylase activity regulating, inhibiting or activating compound in developing new medicines for the prophylaxis and treatment of diseases in mammals, preferably humans, occurring with immune system alterations, preferably with altered T lymphocyte activation; and said regulating compound identification process, and said medicines or pharmaceutical compositions for the prophylaxis and treatment of diseases occurring with T lymphocyte activation alterations. These medicines may be used in a prophylaxis and treatment method for a human being having an immunological disease such as genetic or acquired immunodeficiencies, in vaccination processes, autoimmune and inflammatory diseases.

SEQUENCE LISTING

<110> UNIVERSIDAD AUTONOMA DE MADRID

<120> USO DE LOS COMPUESTOS REGULADORES DE LA ACTIVIDAD TUBULINA
DESACETILASA DE HDAC6 EN LA ELABORACIÓN DE COMPOSICIONES
FARMACÉUTICAS, PROCEDIMIETNO DE IDENTIFICACIÓN DE DICHOS
COMPUESTOS REGULADORES, DICHAS COMPOSICIONES FARMACÉUTICAS Y
SUS APLICACIONES

<130> HDAC6

<160> 4

<170> PatentIn version 3.1

<210> 1
<211> 3648
<212> DNA
<213> Homo sapiens

<220>
<221> CDS
<222> (1)..(3648)
<223> Secuencia codificante de HDAC6

<400> 1
atg acc tca acc ggc cag gat tcc acc aca acc agg cag cga aga agt
48
agg cag aac ccc cag tcg ccc cct cag gac tcc agt gtc act tcg aag
96
cga aat att aaa aag gga gcc gtt ccc cgc tct atc ccc aat cta gcg
144
gag gta aag aag aaa ggc aaa atg aag aag ctc ggc caa gca atg gaa
192
gaa gac cta atc gtg gga ctg caa ggg atg gat ctg aac ctt gag gct
240
gaa gca ctg gct ggc act ggc ttg gtg ttg gat gag cag tta aat gaa
288
ttc cat tgc ctc tgg gat gac agc ttc ccg gaa ggc cct gag cgg ctc
336
cat gcc atc aag gag caa ctg atc cag gag ggc ctc cta gat cgc tgc
384
gtg tcc ttt cag gcc cgg ttt gct gaa aag gaa gag ctg atg ttg gtt
432
cac agc cta gaa tat att gat ctg atg gaa aca acc cag tac atg aat
480
gag gga gaa ctc cgt gtc cta gca gac acc tac gac tca gtt tat ctg
528
cat ccg aac tca tac tcc tgt gcc tgc ctg gcc tca ggc tct gtc ctc
576
agg ctg gtg gat gcg gtc ctg ggg gct gag atc cgg aat ggc atg gcc
624
atc att agg cct cct gga cat cac gcc cag cac agt ctt atg gat ggc
672
tat tgc atg ttc aac cac gtg gct gtg gca gcc cgc tat gct caa cag
720
aaa cac cgc atc cgg agg gtc ctt atc gta gat tgg gat gtg cac cac
768
ggt caa gga aca cag ttc acc ttc gac cag gac ccc agt gtc ctc tat
816
ttc tcc atc cac cgc tac gag cag ggt agg ttc tgg ccc cac ctg aag
864
gcc tct aac tgg tcc acc aca ggt ttc ggc caa ggc caa gga tat acc
912
atc aat gtg cct tgg aac cag gtg ggg atg cgg gat gct gac tac att
960
gct gct ttc ctg cac gtc ctg ctg cca gtc gcc ctc gag ttc cag cct
1008
cag ctg gtc ctg gtg gct gct gga ttt gat gcc ctg caa ggg gac ccc
1056
aag ggt gag atg gcc gcc act ccg gca ggg ttc gcc cag cta acc cac
1104
ctg ctc atg ggt ctg gca gga ggc aag ctg atc ctg tct ctg gag ggt
1152
ggc tac aac ctc cgc gcc ctg gct gaa ggc gtc agt gct tcg ctc cac
1200
acc ctt ctg gga gac cct tgc ccc atg ctg gag tca cct ggt gcc ccc
1248
tgc cgg agt gcc cag gct tca gtt tcc tgt gct ctg gaa gcc ctt gag
1296
ccc ttc tgg gag gtt ctt gtg aga tca act gag acc gtg gag agg gac
1344
aac atg gag gag gac aat gta gag gag agc gag gag gaa gga ccc tgg
1392
gag ccc cct gtg ctc cca atc ctg aca tgg cca gtg cta cag tct cgc
1440
aca ggg ctg gtc tat gac caa aat atg atg aat cac tgc aac ttg tgg
1488
gac agc cac cac cct gag gta ccc cag cgc atc ttg cgg atc atg tgc
1536
cgt ctg gag gag ctg ggc ctt gcc ggg cgc tgc ctc acc ctg aca ccg
1584
cgc cct gcc aca gag gct gag ctg ctc acc tgt cac agt gct gag tac
1632
gtg ggt cat ctc cgg gcc aca gag aaa atg aaa acc cgg gag ctg cac
1680
cgt gag agt tcc aac ttt gac tcc atc tat atc tgc ccc agt acc ttc
1728
gcc tgt gca cag ctt gcc act ggc gct gcc tgc cgc ctg gtg gag gct
1776
gtg ctc tca gga gag gtt ctg aat ggt gct gct gtg gtg cgt ccc cca
1824
gga cac cac gca gag cag gat gca gct tgc ggt ttt tgc ttt ttc aac
1872
tct gtg gct gtg gct gct cgc cat gcc cag act atc agt ggg cat gcc
1920
cta cgg atc ctg att gtg gat tgg gat gtc cac cac ggt aat gga act
1968
cag cac atg ttt gag gat gac ccc agt gtg cta tat gtg tcc ctg cac
2016
cgc tat gat cat ggc acc ttc ttc ccc atg ggg gat gag ggt gcc agc
2064
agc cag atc ggc cgg gct gcg ggc aca ggc ttc acc gtc aac gtg gca
2112
tgg aac ggg ccc cgc atg ggt gat gct gac tac cta gct gcc tgg cat
2160
cgc ctg gtg ctt ccc att gcc tac gag ttt aac cca gaa ctg gtg ctg
2208
gtc tca gct ggc ttt gat gct gca cgg ggg gat ccg ctg ggg ggc tgc
2256
cag gtg tca cct gag ggt tat gcc cac ctc acc cac ctg ctg atg ggc
2304
ctt gcc agt ggc cgc att atc ctt atc cta gag ggt ggc tat aac ctg
2352
aca tcc atc tca gag tcc atg gct gcc tgc act cgc tcc ctc ctt gga
2400
gac cca cca ccc ctg ctg acc ctg cca cgg ccc cca cta tca ggg gcc
2448
ctg gcc tca atc act gag acc atc caa gtc cat cgc aga tac tgg cgc
2496
agc tta cgg gtc atg aag gta gaa gac aga gaa gga ccc tcc agt tct
2544
aag ttg gtc acc aag aag gca ccc caa cca gcc aaa cct agg tta gct
2592
gag cgg atg acc aca cga gaa aag aag gtt ctg gaa gca ggc atg ggg
2640
aaa gtc acc tcg gca tca ttt ggg gaa gag tcc act cca ggc cag act
2688
aac tca gag aca gct gtg gtg gcc ctc act cag gac cag ccc tca gag
2736
gca gcc aca ggg gga gcc act ctg gcc cag acc att tct gag gca gcc
2784
att ggg gga gcc atg ctg ggc cag acc acc tca gag gag gct gtc ggg
2832
gga gcc act ccg gac cag acc acc tca gag gag act gtg gga gga gcc
2880
att ctg gac cag acc acc tca gag gat gct gtt ggg gga gcc acg ctg
2928
ggc cag act acc tca gag gag gct gta gga gga gct aca ctg gcc cag
2976
acc atc tcg gag gca gcc atg gag  gga gcc aca ctg gac  cag act acg
3024
tca gag  gag gct cca ggg ggc  acc gag ctg atc caa  act cct cta
3069
gcc tcg  agc aca gac cac cag  acc ccc cca acc tca  cct gtg cag
3114
gga act  aca ccc cag ata tct  ccc agt aca ctg att  ggg agt ctc
3159
agg acc  ttg gag cta ggc agc  gaa tct cag ggg gcc  tca gaa tct
3204
cag gcc  cca gga gag gag aac  cta cta gga gag gca  gct gga ggt
3249
cag gac  atg gct gat tcg atg  ctg atg cag gga tct  agg ggc ctc
3294
act gat  cag gcc ata ttt tat  gct gtg aca cca ctg  ccc tgg tgt
3339
ccc cat  ttg gtg gca gta tgc  ccc ata cct gca gca  ggc cta gac
3384
gtg acc  caa cct tgt ggg gac  tgt gga aca atc caa  gag aat tgg
3429
gtg tgt  ctc tct tgc tat cag  gtc tac tgt ggt cgt  tac atc aat
3474
ggc cac  atg ctc caa cac cat  gga aat tct gga cac  ccg ctg gtc
3519
ctc agc  tac atc gac ctg tca  gcc tgg tgt tac tac  tgt cag gcc
3564
tat gtc  cac cac cag gct ctc  cta gat gtg aag aac  atc gcc cac
3609
cag aac  aag ttt ggg gag gat  atg ccc cac cca cac  taa
3648


<210>  2
<211>  1215
<212>  PRT
<213>  Homo sapiens

<400>  2

Met Thr Ser Thr Gly Gln Asp Ser Thr Thr Thr Arg Gln Arg Arg Ser
1               5                   10                  15

Arg Gln Asn Pro Gln Ser Pro Pro Gln Asp Ser Ser Val Thr Ser Lys
            20                  25                  30

Arg Asn Ile Lys Lys Gly Ala Val Pro Arg Ser Ile Pro Asn Leu Ala
        35                  40                  45

Glu Val Lys Lys Lys Gly Lys Met Lys Lys Leu Gly Gln Ala Met Glu
    50                  55                  60

Glu Asp Leu Ile Val Gly Leu Gln Gly Met Asp Leu Asn Leu Glu Ala
65                  70                  75                  80

Glu Ala Leu Ala Gly Thr Gly Leu Val Leu Asp Glu Gln Leu Asn Glu
                85                  90                  95

Phe His Cys Leu Trp Asp Asp Ser Phe Pro Glu Gly Pro Glu Arg Leu
            100                 105                 110

His Ala Ile Lys Glu Gln Leu Ile Gln Glu Gly Leu Leu Asp Arg Cys
        115                 120                 125

Val Ser Phe Gln Ala Arg Phe Ala Glu Lys Glu Glu Leu Met Leu Val
    130                 135                 140

His Ser Leu Glu Tyr Ile Asp Leu Met Glu Thr Thr Gln Tyr Met Asn
145                 150                 155                 160

Glu Gly Glu Leu Arg Val Leu Ala Asp Thr Tyr Asp Ser Val Tyr Leu
                165                 170                 175

His Pro Asn Ser Tyr Ser Cys Ala Cys Leu Ala Ser Gly Ser Val Leu
            180                 185                 190

Arg Leu Val Asp Ala Val Leu Gly Ala Glu Ile Arg Asn Gly Met Ala
        195                 200                 205

Ile Ile Arg Pro Pro Gly His His Ala Gln His Ser Leu Met Asp Gly
    210                 215                 220

Tyr Cys Met Phe Asn His Val Ala Val Ala Ala Arg Tyr Ala Gln Gln
225                 230                 235                 240

Lys His Arg Ile Arg Arg Val Leu Ile Val Asp Trp Asp Val His His
                245                 250                 255

Gly Gln Gly Thr Gln Phe Thr Phe Asp Gln Asp Pro Ser Val Leu Tyr
            260                 265                 270

Phe Ser Ile His Arg Tyr Glu Gln Gly Arg Phe Trp Pro His Leu Lys
        275                 280                 285

Ala Ser Asn Trp Ser Thr Thr Gly Phe Gly Gln Gly Gln Gly Tyr Thr
    290                 295                 300

Ile Asn Val Pro Trp Asn Gln Val Gly Met Arg Asp Ala Asp Tyr Ile
305                 310                 315                 320

Ala Ala Phe Leu His Val Leu Leu Pro Val Ala Leu Glu Phe Gln Pro
                325                 330                 335

Gln Leu Val Leu Val Ala Ala Gly Phe Asp Ala Leu Gln Gly Asp Pro
            340                 345                 350

Lys Gly Glu Met Ala Ala Thr Pro Ala Gly Phe Ala Gln Leu Thr His
        355                 360                 365

Leu Leu Met Gly Leu Ala Gly Gly Lys Leu Ile Leu Ser Leu Glu Gly
    370                 375                 380

Gly Tyr Asn Leu Arg Ala Leu Ala Glu Gly Val Ser Ala Ser Leu His
385                 390                 395                 400

Thr Leu Leu Gly Asp Pro Cys Pro Met Leu Glu Ser Pro Gly Ala Pro
                405                 410                 415

Cys Arg Ser Ala Gln Ala Ser Val Ser Cys Ala Leu Glu Ala Leu Glu

            420                 425                 430

Pro Phe Trp Glu Val Leu Val Arg Ser Thr Glu Thr Val Glu Arg Asp
        435                 440                 445

Asn Met Glu Glu Asp Asn Val Glu Glu Ser Glu Glu Gly Pro Trp
    450                 455                 460

Glu Pro Pro Val Leu Pro Ile Leu Thr Trp Pro Val Leu Gln Ser Arg
465 470 475 480

Thr Gly Leu Val Tyr Asp Gln Asn Met Met Asn His Cys Asn Leu Trp
485 490 495

Asp Ser His His Pro Glu Val Pro Gln Arg Ile Leu Arg Ile Met Cys
                500                 505                 510

Arg Leu Glu Glu Leu Gly Leu Ala Gly Arg Cys Leu Thr Leu Thr Pro
            515                 520                 525

Arg Pro Ala Thr Glu Ala Glu Leu Leu Thr Cys His Ser Ala Glu Tyr
        530                 535                 540

Val Gly His Leu Arg Ala Thr Glu Lys Met Lys Thr Arg Glu Leu His
545 550 555 560

Arg Glu Ser Ser Asn Phe Asp Ser Ile Tyr Ile Cys Pro Ser Thr Phe
565 570 575

Ala Cys Ala Gln Leu Ala Thr Gly Ala Ala Cys Arg Leu Val Glu Ala
                580                 585                 590

Val Leu Ser Gly Glu Val Leu Asn Gly Ala Ala Val Val Arg Pro Pro
            595                 600                 605

Gly His His Ala Glu Gln Asp Ala Ala Cys Gly Phe Cys Phe Phe Asn
        610                 615                 620

Ser Val Ala Val Ala Ala Arg His Ala Gln Thr Ile Ser Gly His Ala
625 630 635 640

Leu Arg Ile Leu Ile Val Asp Trp Asp Val His His Gly Asn Gly Thr
645 650 655

Gln His Met Phe Glu Asp Asp Pro Ser Val Leu Tyr Val Ser Leu His
                660                 665                 670

Arg Tyr Asp His Gly Thr Phe Phe Pro Met Gly Asp Glu Gly Ala Ser
            675                 680                 685

Ser Gln Ile Gly Arg Ala Ala Gly Thr Gly Phe Thr Val Asn Val Ala
        690                 695                 700

Trp Asn Gly Pro Arg Met Gly Asp Ala Asp Tyr Leu Ala Ala Trp His
705 710 715 720

Arg Leu Val Leu Pro Ile Ala Tyr Glu Phe Asn Pro Glu Leu Val Leu
                725                 730                 735

Val Ser Ala Gly Phe Asp Ala Ala Arg Gly Asp Pro Leu Gly Gly Cys
            740                 745                 750

Gln Val Ser Pro Glu Gly Tyr Ala His Leu Thr His Leu Leu Met Gly
        755                 760                 765

Leu Ala Ser Gly Arg Ile Ile Leu Ile Leu Glu Gly Gly Tyr Asn Leu
    770                 775                 780

Thr Ser Ile Ser Glu Ser Met Ala Ala Cys Thr Arg Ser Leu Leu Gly
785                 790                 795                 800

Asp Pro Pro Pro Leu Leu Thr Leu Pro Arg Pro Pro Leu Ser Gly Ala
                805                 810                 815

Leu Ala Ser Ile Thr Glu Thr Ile Gln Val His Arg Arg Tyr Trp Arg
            820                 825                 830

Ser Leu Arg Val Met Lys Val Glu Asp Arg Glu Gly Pro Ser Ser Ser
        835                 840                 845

Lys Leu Val Thr Lys Lys Ala Pro Gln Pro Ala Lys Pro Arg Leu Ala
    850                 855                 860

Glu Arg Met Thr Thr Arg Glu Lys Lys Val Leu Glu Ala Gly Met Gly
865                 870                 875                 880

Lys Val Thr Ser Ala Ser Phe Gly Glu Glu Ser Thr Pro Gly Gln Thr
                885                 890                 895

Asn Ser Glu Thr Ala Val Val Ala Leu Thr Gln Asp Gln Pro Ser Glu
            900                 905                 910

Ala Ala Thr Gly Gly Ala Thr Leu Ala Gln Thr Ile Ser Glu Ala Ala
        915                 920                 925

Ile Gly Gly Ala Met Leu Gly Gln Thr Thr Ser Glu Glu Ala Val Gly
    930                 935                 940

Gly Ala Thr Pro Asp Gln Thr Thr Ser Glu Glu Thr Val Gly Gly Ala
945                 950                 955                 960

Ile Leu Asp Gln Thr Thr Ser Glu Asp Ala Val Gly Gly Ala Thr Leu
                965                 970                 975

Gly Gln Thr Thr Ser Glu Glu Ala Val Gly Gly Ala Thr Leu Ala Gln
            980                 985                 990

Thr Ile Ser Glu Ala Ala Met Glu  Gly Ala Thr Leu Asp  Gln Thr Thr
        995                 1000                1005

Ser Glu  Glu Ala Pro Gly Gly  Thr Glu Leu Ile Gln  Thr Pro Leu
    1010                1015                1020

Ala Ser Ser Thr Asp His Gln Thr Pro Pro Thr Ser Pro Val Gln
    1025                1030                1035

Gly Thr Thr Pro Gln Ile Ser Pro Ser Thr Leu Ile Gly Ser Leu
    1040                1045                1050

Arg Thr Leu Glu Leu Gly Ser Glu Ser Gln Gly Ala Ser Glu Ser
    1055                1060                1065

Gln Ala Pro Gly Glu Glu Asn Leu Leu Gly Glu Ala Ala Gly Gly
    1070                1075                1080

Gln Asp Met Ala Asp Ser Met Leu Met Gln Gly Ser Arg Gly Leu
    1085                1090                1095

Thr Asp Gln Ala Ile Phe Tyr Ala Val Thr Pro Leu Pro Trp Cys
    1100                1105                1110

Pro His Leu Val Ala Val Cys Pro Ile Pro Ala Ala Gly Leu Asp
    1115                1120                1125

Val Thr Gln Pro Cys Gly Asp Cys Gly Thr Ile Gln Glu Asn Trp
    1130                1135                1140

Val Cys Leu Ser Cys Tyr Gln Val Tyr Cys Gly Arg Tyr Ile Asn
    1145                1150                1155

Gly His Met Leu Gln His His Gly Asn Ser Gly His Pro Leu Val
    1160                1165                1170

Leu Ser Tyr Ile Asp Leu Ser Ala Trp Cys Tyr Tyr Cys Gln Ala
    1175                1180                1185

Tyr Val His His Gln Ala Leu Leu Asp Val Lys Asn Ile Ala His
    1190                1195                1200

Gln Asn Lys Phe Gly Glu Asp Met Pro His Pro His
    1205                1210                1215


<210>  3
<211>  26
<212>  DNA
<213>  Artificial Sequence

<220>
<223>  Oligo HDAC6 5´

<400>  3
cggcgtcgac gatgacctca accggc
26


<210>  4
<211>  37

<212>  DNA
<213>  Artificial Sequence

<220>
<223>  Oligo HDAC6 3´

<400>  4
cggccaccgg tgggtgtggg tggggcatat cctcccc
37

## Description

### OBJECT OF THE INVENTION

The object of the present invention is the use of an HDAC6 protein tubulin deacetylase activity regulating, inhibiting or activating compound for developing new medicines for the prophylaxis and treatment of mammal diseases, preferably human, occurring with immune system alterations, preferably with altered T lymphocyte activation; as well as an identification process for said regulating compounds and said medicines or pharmaceutical compositions for the prophylaxis and treatment of diseases occurring with altered T lymphocyte activation. These medicines may be used in a prophylaxis and treatment method for a mammal, preferably a human, having an immunological disease such as genetic or acquired immunodeficiencies, in vaccination processes, autoimmune and inflammatory diseases.

### FIELD OF THE ART

Acting on the functional activities of HDAC6 tubulin deacetylase or other enzymes regulating tubulin acetylation/deacetylation status as immune regulators may promote the development of new treatments for readjusting the immune response, including negative regulation in the case of autoimmune diseases and allergies, or positive in the case of immunodeficiency therapies. Development of these methods is based on regulating tubulin acetylation levels using HDAC6 tubulin deacetylase as a therapeutic target. It must therefore be part of the pharmacological and biomedical sector for its use in the health sector, and more specifically to the field of identifying new therapeutic compounds and pharmaceutical compositions for the prophylaxis and treatment of diseases or disorders occurring with immune system alterations.

### STATE OF THE ART

Pharmacological treatment of diseases with an immune pathogenic component is a field of intense study, this category including autoimmune diseases affecting about 5% of the world population (O'Shea, J.J., Ma, A., and Lipsky, P. (2002). Cytokines and autoimmunity. Nat. Rev. Immunol. 2, 37-45), allergies, chronic and infectious inflammatory diseases and tumor processes which are a major cause of mortality in industrialized countries. Therefore, regulating the immune response is crucial in treating the most important causes of morbimortality in our society.

T cell activation requires binding of the antigen duly presented by major histocompatibility complex molecules to antigen-presenting cells. This intercellular binding has been called immunological synapse (Bromley, S.K., Burack, W.R., Johnson, K.G., Somersalo, K., Sims, T.N., Sumen, C., Davis, M.M., Shaw, A.S., Allen, P.M., and Dustin, M.L. (2001). The immunological synapse. Annu. Rev. Immunol. 19, 375-396) and is characterized by being organized in two supramolecular activation receptor clusters : i) a central cluster where the TcR complex and costimulating molecules such as CD4, CD2 and CD28 are concentrated (Monks, C.R., Friberg, B.A., Kupfer, H., Sciaky, N., and Kupfer, A. (1998). Three-dimensional segregation of supramolecular activation clusters in T cells. Nature 395, 82-86; Grakoui, A., Bromley, S.K., Sumen, C., Davis, M.M., Shaw, A.S., Allen, P.M., and Dustin, M.L. (1999). The immunological synapse: a molecular machine controlling T cell activation. Science 285, 221-227), and ii) a peripheral ring of adhesion receptors containing *inter alia* leukocyte integrin LFA-1 (Monks, C.R., Friberg, B.A., Kupfer, H., Sciaky, N., and Kupfer, A. (1998). Three-dimensional segregation of supramolecular activation clusters in T cells. Nature 395, 82-86). T cell activation by the antigen also implies translocating the microtubule organizing centre (MTOC) and the Golgi apparatus towards the contact site with the antigen-presenting cell as well as IL-2 production (Geiger, B., Rosen, D., and Berke, G. (1982). Spatial relationships of microtubule-organizing centers and the contact area of cytotoxic T lymphocytes and target cells. J. Cell Biol. 95, 137-143; Ryser, J., Rungger-Brändle, E., Chaponier, C., Gabbiani, J., and Vassalli, P. (1982). The area of attachment of cytotoxic T lymphocytes to their target cells shows high motility and polarization of actin, but not myosin. J. Immunol. 128, 1159-1162; Kupfer, A., Swain, S.L., Janeway, C.A., and Singer, S.J. (1986). The specific direct interaction of helper T cells and antigen-presenting B cells. Proc. Natl. Acad. Sci. USA. 83, 6080-6083).

It is known that actin filaments and associated proteins play an important role in immunological synapse formation, organizing the arrangement of the receptors for the antigen+ and the adhesion molecules (Monks, C.R., Friberg, B.A., Kupfer, H., Sciaky, N., and Kupfer, A. (1998). Three-dimensional segregation of supramolecular activation clusters in T cells. Nature 395, 82-86; Allenspach, E.J., Cullinan, P., Tong, J., Tang, Q., Tesciuba, A.G., Cannon, J.L., Takahashi, S.M., Morgan, R., Burkhardt, J.K., and Sperling, A.I. (2001); Delon, J., Kaibuchi, K., and Germain, R. (2001). Exclusion of CD43 from the immunological synapse is mediated by phosphorylation-regulated relocation of the cytoskeletal adaptor moesin. Immunity 15, 691-701; Roumier, A., Olivo-Marin, J.C., Arpin, M., Michel, F., Martin, M., Mangeat, P., Acuto, O., Dautry-Varsat, A., and Alcover, A. (2001). The membrane-microfilament linker ezrin is involved in the formation of the immunological synapse and it T cell activation. Immunity 15, 715-728). However, to date, little was known of the role played by microtubules in the development of the immune response (Nogales, E. (2001). Structural insights into microtubule function. Annu. Rev. Biophys. Biomol. Struct. 30, 397-420).

In the present invention it is demonstrated that acetylation of the ε-amino groups of lysine 40 of the α tubulin subunit in microtubules (Thompson, W.C., Asai, D.J., and Carney, D.H. (1984). Heterogeneity among microtubules of the cytoplasmatic microtubule complex detected by a monoclonal antibody to alpha tubulin. J. Cell Biol., 98 1017-1025; L'Hernault, S.W., and Rosenbaum, J.L. (1983). Chlamydomonas α-tubulin is post-translationally modified by acetylation on the ε-amino group of a lysin. Biochemistry 24, 473-478; Piperno, G., LeDizet, M., and Chang, X. (1987). Microtubules containing acetylated α-tubulin in mammalian cells in culture. J. Cell Biol. 104, 289-302) plays an important role in antigen-dependent T cell activation.

The deacetylating activity of histone deacetylase (HDAC6) microtubules has been recently identified (Hubbert, C., Guardiola, A., Shao, R., Kawaguchi, Y., Ito, A., Nixon, A., Yoshida, M., Wang, X., and Yao, T. (2002). HDAC6 is a microtubule-associated deacetylase. Nature 417, 455-458; Matsuyama, A., Shimazu, T., Sumida, Y., Saito, A., Seigneurin-Berny, D., Osada, H., Komatsu, Y., Nishino, N., Khochbin, S., Horinouchi, S., and Yoshida, M. (2002). In vivo destabilization of dynamic microtubules by HDAC6-mediated deacetylation. EMBO J. 21, 6820-6831). HDAC6 is the only known member of the class II histone deacetylase family capable of deacetylating histones and tubulin (Grozinger, C., Hassing, C., and Schreiber, S.L. (1999). Three proteins define a class of human histone deacetylases related to yeast Hda1p. Proc. Natl. Acad. Sci. USA 96, 4868-4873; Haggarty, S.J., Koeller, K.M., Wong, J.C., Grozinger, C., and Schreiber, S.L. (2003). Domain selective small-molecule inhibitor of histone deacetylase 6 (HDAC6)-mediated tubulin deacetylation. Proc. Natl. Acad. Sci. USA 100, 4389-4394). This enzyme deacetylases tubulin in the cells and its activity may be inhibited by the pharmacological agents trichostatin A and tubacin (Hubbert, C., Guardiola, A., Shao, R., Kawaguchi, Y., Ito, A., Nixon, A., Yoshida, M., Wang, X., and Yao, T. (2002). HDAC6 is a microtubule-associated deacetylase. Nature 417, 455-458; Haggarty, S.J., Koeller, K.M., Wong, J.C., Grozinger, C., and Schreiber, S.L. (2003). Domain selective small-molecule inhibitor of histone deacetylase 6 (HDAC6)-mediated tubulin deacetylation. Proc. Natl. Acad. Sci. USA 100, 4389-4394).

On the other hand the wide range of cellular activities appearing in the state of the art in which HDACs are involved and which have allowed establishing new therapeutic approaches to multiple human pathologies by means of the use of HDAC inhibitors and agonists and the development of new identification and evaluation methods for new therapeutic compounds, must be stressed. The following applications have been described in this sense as therapeutic target proteins derived from the functional activities they participate in:
- target protein for treating cancer due to the ability of these inhibitors to induce tumor cell apoptosis, their ability to promote said cell differentiation, or the ability to induce expression in said antigen cells promoting a greater immune response (Marks, PA, Fichan, VM, Breslow, R, and Rifkind, RA. (2001). Histone deacetylase as new cancer drugs. Curr. Opin. Oncol. 13:477-489; US patent 6,262,116, Transcription therapy for cancers; US patent 6,387,673, Compounds useful for the modulation of processes mediated by nuclear hormone receptors, methods for the identification and use of such compounds; US patent 6,399,568, Cyclic tetrapeptide derivatives and medicinal use thereof; WO patent 02/15921, Methods of treating cutaneous T-cell lymphoma and peripheral T-cell lymphoma (unspecified) by administering a histone deacetylase inhibitor; WO patent 02/055688, Histone deacetylase inhibitors in diagnosis and treatment of thyroid neoplasms; US patent 6,071,923, Retinoyloxy aryl-substituted alkylene butyrates useful for the treatment of cancer and other proliferative diseases), and for treating other proliferative diseases (PCT application of US patent 20020161045, Novel acetyloxymethyl esters and methods for using the same),
- target protein for treating diseases caused by protozoa (Kwon HJ, Kim JH, Kim M, Lee JK, Hwang WS, Kim DY (2003). Antiparasitic activity of depudecin on Neospora caninum via the inhibition of histone deacetylase Vet. Parasitol. 112, 269-276; US patent 6,428,983, Histone deacetylase as target for antiprotozoal agents; PCT application for US patent 20030078369, Apicidin-derived cyclic tetrapeptides),
- target protein for treating cardiac hypertrophy (van Zonneveld AJ (2003). Molecular biology and genetics in cardiovascular research: highlights of 2002. Neth J Med. 61, 28-34; PCT application for US patent 20030114340, Inhibition of histone deacetylase as a treatment for cardiac hypertrophy; US patent 6,632,628, Methods and compositions relating to HDAC 4 and 5 regulation of cardiac gene expression),
- target protein for treating diseases or disorders affecting connective tissue (Xu RH, Peng Y, Fan J, Yan D, Yamagoe S, Princler G, Sredni D, Ozato K, Kung HF (2000). Histone acetylation is a checkpoint in FGF-stimulated mesoderm induction. Dev Dyn. 218, 628-35; PCT application for US patent 20030206946, Methods for therapy of connective tissue disease),
- target protein for treating spinal muscular atrophy (US patent 6,376,508, Treatments for spinal muscular atrophy),
- target protein for treating central nervous system neurodegenerative diseases such as Alzheimer's disease, multiple sclerosis and amyotrophic lateral sclerosis (Hoshino M, Tagawa K, Okuda T, Murata M, Oyanagi K, Arai N, Mizutani T, Kanazawa I, Wanker EE, Okazawa H (2003). Histone deacetylase activity is retained in primary neurons expressing mutant huntingtin protein. J Neurochem. 87, 257-267; Hockly E, Richon VM, Woodman B, Smith DL, Zhou X, Rosa E, Sathasivam K, Ghazi-Noori S, Mahal A, Lowden PA, Steffan JS, Marsh JL, Thompson LM, Lewis CM, Marks PA, Bates GP (2003). Suberoylanilide hydroxamic acid, a histone deacetylase inhibitor, ameliorates motor deficits in a mouse model of Huntington's disease. Proc Natl Acad Sci USA. 100, 2041-6; WO 03/083067, Histone deacetylase inhibitors for the treatment of multiple sclerosis, amyotrophic lateral sclerosis and Alzheimer diseases; WO 03/080864, Histone deacetylase: novel molecular target of neurotoxicity; WO 03/032921, Treatment of neurodegenerative diseases and cancer of the brain),
- key target protein in regeneration and differentiation processes of multiple cell types (Zhang CL, McKinsey TA, Olson EN (2002). Association of class II histone deacetylases with heterochromatin protein 1: potential role for histone methylation in control of muscle differentiation. Mol Cell Biol. 22, 7302-7312; WO 03/033678, Methods of using deacetylase inhibitors to promote cell differentiation and regeneration; WO 0023567, Promotion of self-renewal and improved gene transduction of hematopoietic stem cells by histone deacetylase inhibitors), and
- target protein for regulating signal transmission through receptors, particularly as an enhancer of the bronchodilator effect of corticoids in treating asthma, in this case by means of HDAC protein agonists (Ito K, Caramori G, Lim S, Oates T, Chung KF, Barnes PJ, Adcock IM (2002). Expression and activity of histone deacetylases in human asthmatic airways. Am J Respir Crit Care Med. 166, 392-396; PCT application for US patent 20030134865).

Actin microfilaments are considered the most important cytoskeletal components for T cell activation (Valitutti S.M., Dessing, M., Aktories, K., Gallati, H., and Lanzavecchia, A. (1995). Sustained signaling leading to T cell activation results from prolonged T cell receptor occupancy. J. Exp. Med. 181, 577-584; Serrador J.M., Nieto, M., and Sánchez-Madrid, F. (1999). Cytoskeletal rearrangement during migration and activation of T lymphocytes. Trends Cell Biol. 9, 228-232; Dustin, M.L., and Cooper, J.A. (2000). The immunological synapse and the actin cytoskeleton: molecular hardware for T cell signaling. Nat. Immunol. 1, 23-29). However, increasing evidence is found that other cytoskeletal proteins participate in this process, although until now microtubule acetylation has not been related to any immune pathology. Therefore, regulation of HDAC6-mediated tubulin deacetylating activity and its implications in modulating T lymphocyte activation described in this document for the first time may result in an effective treatment in diseases occurring with an exacerbated immune response and also in those occurring with a deficient immune response.

### DESCRIPTION OF THE PATENT

### Brief Description

An object of the invention is the use of an HDAC6 protein tubulin deacetylase activity regulating, inhibiting or activating compound in developing medicines or pharmaceutical compositions for the prophylaxis and treatment of diseases in mammals, and preferably in humans, occurring with immune system alterations, preferably with altered T lymphocyte activation.

Another object of the present invention is a process of identifying and evaluating the activity of HDAC6 protein regulating compounds on T lymphocyte activation disorders comprising the following steps:
i) the contact of preferably human T lymphocytes with the potential compound object of this process and incubation in suitable conditions,
ii) determination of a parameter indicating tubulin acetylation levels in the T lymphocyte of i) or of any other parameter related to the T lymphocyte activation process in which the HDAC6 protein participates, and
iii) identification of an HDAC6 protein activity inhibiting or agonist compound when an increase in tubulin acetylation and/or a parameter indicating T lymphocyte activation is observed, or when a reduction in tubulin acetylation and/or a parameter indicating T lymphocyte inactivation is observed, respectively.

Another object of the present invention is a pharmaceutical composition or medicine for the prophylaxis and treatment of diseases, disorders or pathologies occurring with immune system alterations, preferably with altered T lymphocyte activation, comprising a therapeutically effective amount of an HDAC6 protein tubulin deacetylase activity regulating compound, optionally together with one or more adjuvants and/or pharmaceutically acceptable vehicles, and which is able to regulate said altered T lymphocyte activation.

Finally, another object of the present invention is the use of the pharmaceutical composition of the present invention in a prophylaxis and treatment method for a mammal, preferably a human being, having a disease, disorder or pathology characterized by an undesirable immune response, consisting in the administration of said therapeutic composition modifying the tubulin acetylation status and acting as an immunomodulatory composition.

### Detailed Description

The results of the present invention show that microtubules also play an important role in early and late T cell activation. Antigen binding to the T cell receptor modifies microtubule acetylation in a dynamic manner in which they are initially deacetylated so that about 15 minutes later they are progressively reacetylated. The initial deacetylation step occurs at the same time as T cell receptor activation, reinforcing the hypothesis that the initial deacetylation step is induced through the T cell receptor. The fact that initial deacetylation inhibition by means of treatment with trichostatin A, an HDAC6 tubulin deacetylase activity inhibitor (Example 3), favors early activation events and, in contrast, HDAC6 tubulin deacetylase activity activation hinders them (Example 3) shows that the dynamic microtubule acetylation/deacetylation process is required for the correct organization of membrane receptors in the immunological synapse and the resulting T cell activation. In cell conjugates between cooperating T cells directing the immune response and antigen-presenting cells, the microtubules are acetylated in the contact site between the T cell and the antigen-presenting cell in the immunological synapse taking place in T lymphocyte activation. It is also shown in this invention that HDAC6 tubulin deacetylase is concentrated in the immunological synapse and that its overexpression produces its disorganization. Furthermore, uncontrolled HDAC6 activation inhibits the secretory apparatus translocation towards the antigen-presenting cell and reduces IL-2 production, which are, respectively, two early and late activation events. The data thus suggests the use of the microtubule acetylation/deacetylation equilibrium (through HDAC6, for example) as a new therapeutic target in diseases with immune participation.

In short, the present invention is based on the fact that microtubule acetylation is important for modulating T cell activation and is regulated by the HDAC6 protein, which allows defining new therapeutic approaches for the prophylaxis and treatment of diseases occurring with immune system alterations. These therapeutic approaches are based on the use of said HDAC6 protein activity regulating, inhibiting or activating compounds or agents.

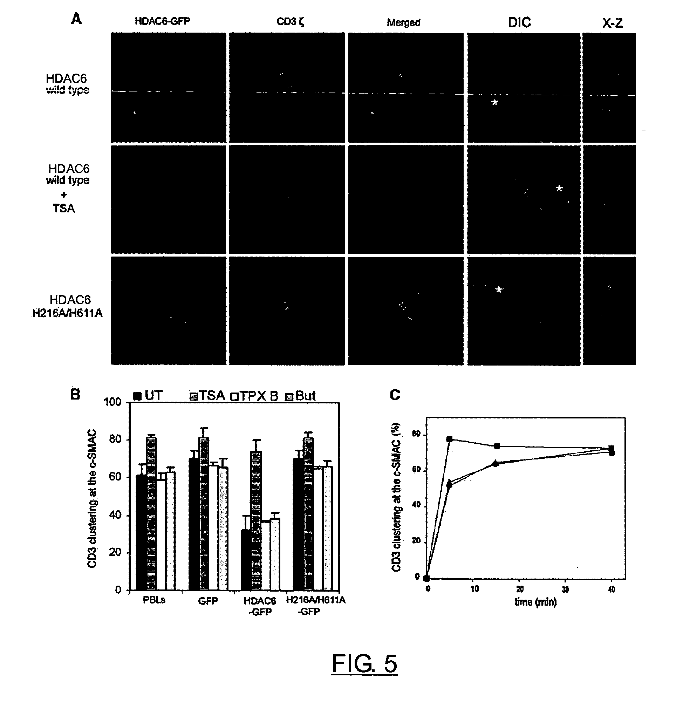

HDAC6 inhibiting or antagonist compounds, which consequently produce a net tubulin acetylation, are useful for restoring a deficient immune response. Thus, pretreating T cells with the HDAC6 inhibitor trichostatin A reverts the immunological synapse disorganization as well as the Microtubule Organizing Centre (MTOC) translocation inhibition occurring due to HDAC6 overexpression (Figures 5-6 and 7, respectively). On the other hand, activating or agonist HDAC6 compounds could perform a role in reducing damage associated to an exacerbated immune response, such as those occurring in an autoimmune disease. In this sense, gene transfer technology could, by means of viral vectors, be useful in gene therapy for expressing the HDAC6 enzyme in a tissue-specific manner in T lymphocytes of patients with this type of diseases.

Thus, an object of the present invention is the use of an HDAC6 protein activity regulating, inhibiting or activating compound or agent, hereinafter the use of a compound of the present invention, in developing medicines or pharmaceutical compositions for the prophylaxis and treatment of diseases, disorders or pathologies in mammals, preferably humans, occurring with immune system alterations, preferably with altered T lymphocyte activation.

A particular object of the present invention is the use of an HDAC6 protein activity regulating compound in which the regulating compound used in developing medicines or therapeutic compositions for prophylaxis and treatment of diseases, disorders or pathologies in mammals, preferably humans, occurring with immune system alterations, is an HDAC6 protein activity inhibitor or antagonist inducing T lymphocyte activation.

The term "inhibiting or antagonist compound/agent" as used in the present invention refers to a molecule which, when binding or interacting with the HDAC6 protein (SEQ ID NO. 2) or with functional fragments thereof, reduces or eliminates the intensity or duration of the biological activity of said protein. This definition also includes those compounds preventing or reducing the expression of the gene coding for the HDAC6 protein, that is, preventing or reducing gene transcription, mRNA maturation, mRNA translation and post-translational modification. An inhibiting agent may be made up of a peptide, a protein, a nucleic acid or polynucleotide, a carbohydrate, an antibody, a chemical compound or any other type of molecule reducing or eliminating the effect and/or function of the HDAC6 protein tubulin deacetylase.

In an illustrative manner, said polynucleotide may be a polynucleotide coding for a specific HDAC6 protein gene or mRNA antisense nucleotide sequence or a polynucleotide coding for a specific HDAC6 protein mRNA ribozyme, or a polynucleotide coding for a specific HDAC6 protein mRNA aptamer, or a polynucleotide coding for a specific HDAC6 protein mRNA interference RNA (RNAi).

These mentioned polynucleotides may be used in a gene therapy process in which integration thereof in the cells of a human patient is allowed by means of any technique or process. This object may be achieved by means of administering a gene construct to these cells comprising one of the mentioned polynucleotides so as to transform said cells, allowing their expression within the same, such that HDAC6 protein expression is inhibited. Said gene construct may advantageously be included in a vector such as, for example, an expression vector or a transfer vector.

The term "vector" as used in the present invention refers to systems used in the process of transferring an exogenous gene or an exogenous gene construct to the interior of the cell, thus allowing stable transport of exogenous genes and gene constructs. Said vectors may be non-viral vectors or viral vectors (Pfeifer A, Verma IM (2001) Gene therapy: promises and problems. Annu Rev Genomics Hum Genet 2: 177-211) and their administration may be prepared by a person skilled in the art according to the requirements and specificities of each case.

Therefore, in another particular embodiment of the invention said use of a compound is based on the fact that the inhibiting compound is a nucleic acid or a polynucleotide preventing or reducing the expression of the gene coding for the human HDAC6 protein and including a nucleotide sequence selected from:
a) an antisense nucleotide sequence specific to the HDAC6 protein gene or mRNA sequence,
b) a ribozyme specific to HDAC6 protein mRNA,
c) an aptamer specific to HDAC6 protein mRNA, and
d) an interference RNA (RNAi) specific to HDAC6 protein mRNA.

Nucleotide sequences a)-d) mentioned above prevent the expression of the gene in mRNA or of the mRNA in the HDAC6 protein, and therefore repress its biological function, and may be developed by a person skilled in the art of genetic engineering according to the existing knowledge in the state of the art on transgenesis and gene expression repression (Clarke, A.R. (2002) Transgenesis Techniques. Principles and Protocols, 2nd Ed. Humana Press, Cardiff University; US patent 20020128220. Gleave, Martin. TRPM-2 antisense therapy; Puerta-Ferández E et al. (2003) Ribozymes: recent advances in the development of RNA tools. FEMS Microbiology Reviews 27: 75-97; Kikuchi et al., 2003. RNA aptamers targeted to domain II of Hepatitis C virus IRES that bind to its apical loop region. J. Biochem. 133, 263-270; Reynolds A. et al., 2004. Rational siRNA design for RNA interference. Nature Biotechnology 22 (3): 326-330).

On the other hand, there are only a few HDAC6 protein tubulin deacetylase activity modifying compounds disclosed in the state of the art which can be used in the scope of the present invention, and which are indicated below with an illustrative character and without this implying a limitation to the scope of the present invention on current and future HDAC6 inhibitors: hydroxamic acid derivative (trichostatin A (TSA) (Richon CM et al., 2001 Blood Cells Mol dis 27(1); 260-4, tubacin (Haggarty, S.J., Koeller, K.M., Wong, J.C., Grozinger, C., and Schreiber, S.L., (2003). Domain selective small-molecule inhibitor of histone deacetylase 6 (HDAC6)-mediated tubulin deacetylation. Proc. Natl. Acad. Sci. USA 100, 4389-4394), and HDAC6 protein mRNA antisense oligonucleotides (patent WO 03/006652, Inhibition of specific histone deacetylase isoforms). In the framework of the present invention the special role of those HDAC6 protein inhibiting compounds must be stressed, and more specifically those compounds which, like tubacin and its derivatives, are able to bind to and block its only catalytic domain which deacetylates tubulin in a specific manner (Haggarty, S.J., Koeller, K.M., Wong, J.C., Grozinger, C., and Schreiber, S.L., (2003). Domain selective small-molecule inhibitor of histone deacetylase 6 (HDAC6)-mediated tubulin deacetylation. Proc. Natl. Acad. Sci. USA 100, 4389-4394).

Another particular object of the present invention is the use of an HDAC6 protein tubulin deacetylase activity regulating compound of the invention, in which the regulating compound used in developing medicines or pharmaceutical compositions for the prophylaxis and treatment of diseases, disorders or pathologies in mammals, preferably humans, occurring with immune system alterations, is an HDAC6 protein tubulin deacetylase activity activator, inductor or agonist inhibiting T lymphocyte activation.

The term "activating or agonist compound/agent" as used in the present invention refers to a molecule which, when binding or interacting with the HDAC6 protein or with functional fragments thereof, increases the intensity or extends the duration of the biological activity of said protein. This definition further includes those compounds that allow increasing the expression of the gene coding for the HDAC6 protein. An activating agent may be made up of a peptide, a protein, a nucleic acid, carbohydrates, an antibody, a chemical compound or any other type of molecule increasing the effect and/or the duration of HDAC6 protein activity. There are HDAC6 protein activity agonist compounds disclosed in the state of the art which may be used in the scope of the present invention as HDAC6 protein agonists, and which are indicated below with an illustrative character and without this implying a limitation to the scope of the present invention: xanthines (US patent 20030134865).

Another particular aspect of the present invention is the use of an agonist compound for developing a medicine or therapeutic composition for the prophylaxis and treatment of a disease occurring with an immune system exacerbation, based on the compound being made up of a gene construct containing a coding nucleotide sequence (SEQ ID NO. 1) allowing expression of the human HDAC6 protein (SEQ ID NO. 2) inside human cells, preferably in T lymphocytes, in a similar manner to the gene constructs developed in the present invention (see examples 3, 4 and 5). In this sense, there may be other nucleotide sequences coding a peptide or protein with HDAC6 protein tubulin deacetylase activity which may be obtained by a person skilled in the art, as well as the human HDAC6 nucleotide sequence (SEQ ID NO. 1), for example, fragments thereof maintaining said biological activity or complete or fragment sequences homologous to both forms from other species, preferably eukaryotic animals similar to the human species.

Therefore, a particular embodiment is the use of an agonist compound in developing a medicine or therapeutic compound for the prophylaxis and treatment of a disease occurring with an immune system aggravation, based on the fact that the compound is made up of a gene construct or expression vector allowing expression of a peptide or protein inside human cells, preferably in T lymphocytes, mimicking or reproducing the human HDAC6 protein tubulin deacetylase biological activity and comprising a nucleotide sequence belonging to the following group:
a) a nucleotide sequence (SEQ ID NO. 1) coding for human HDAC6 protein (SEQ ID NO. 2),
b) a fragment of the nucleotide sequence of a), and
c) a nucleotide sequence similar to the sequences of a) and b).

The term "similar" in the sense used in this description, intends to include any nucleotide sequence which may be isolated or constructed based on the nucleotide sequences shown in the present specification, for example, by means of introducing conservative or non-conservative substitutions, including inserting one or more nucleotides, adding one or more nucleotides on any of the molecule ends or deleting one or more nucleotides on any end or inside the sequence, and allowing expression of a peptide with human HDAC6 protein tubulin deacetylase activity.

Generally, a similar amino acid sequence is substantially homologous to the previously mentioned nucleotide sequence. The term "substantially homologous" in the sense used in this description means that the nucleotide sequences in question have a degree of identity of at least 40%, preferably of at least 85%, or more preferably of at least 95%.

As has been previously mentioned, these constructs and vectors may be developed by a person skilled in the art by means of information existing in the state of the art and provided in the present specification (Sambroock et al., Molecular Cloning; A Laboratory Manual, Second Edition, Cold Spring Harbor Press. Cold Spring Harbor, NY (1989)).

On the other hand, the origin of these HDAC6 protein tubulin deacetylase activity regulating, inhibiting or agonist compounds can be varied, so that they can be of natural origin (for example, of plant, bacterial, viral, animal or eukaryotic microorganism origin) or of synthetic origin.

An additional object of the present invention is a process of identifying and evaluating the activity of HDAC6 protein regulating compounds on T lymphocyte activation, hereinafter compound identification process of the present invention, comprising the following steps:
i) the contact of preferably human T lymphocytes with the potential compound object of this process and incubation in suitable conditions,
ii) determination of a parameter indicating tubulin acetylation levels in the T lymphocyte of i) or of any other parameter related to the T lymphocyte activation process in which the HDAC6 protein participates, and
iii) identification of an HDAC6 protein activity inhibiting or agonist compound when an increase in tubulin acetylation and/or a parameter indicating T lymphocyte activation is observed, or when a reduction in tubulin acetylation and/or a parameter indicating T lymphocyte inactivation is observed, respectively.

A particular object of the present invention is a potential inhibiting compound identification process of the invention where the T lymphocytes of point i) can be stimulated or prepared by means of processes known in the state of the art (see Examples 1-5; stimulation with antigen-presenting cells (Raji cells) plus SEE and transformation of T lymphocyte cell cultures with a gene construct allowing expression of the HDAC6 gene) allowing a better evaluation of the effect of said potential inhibiting compounds.

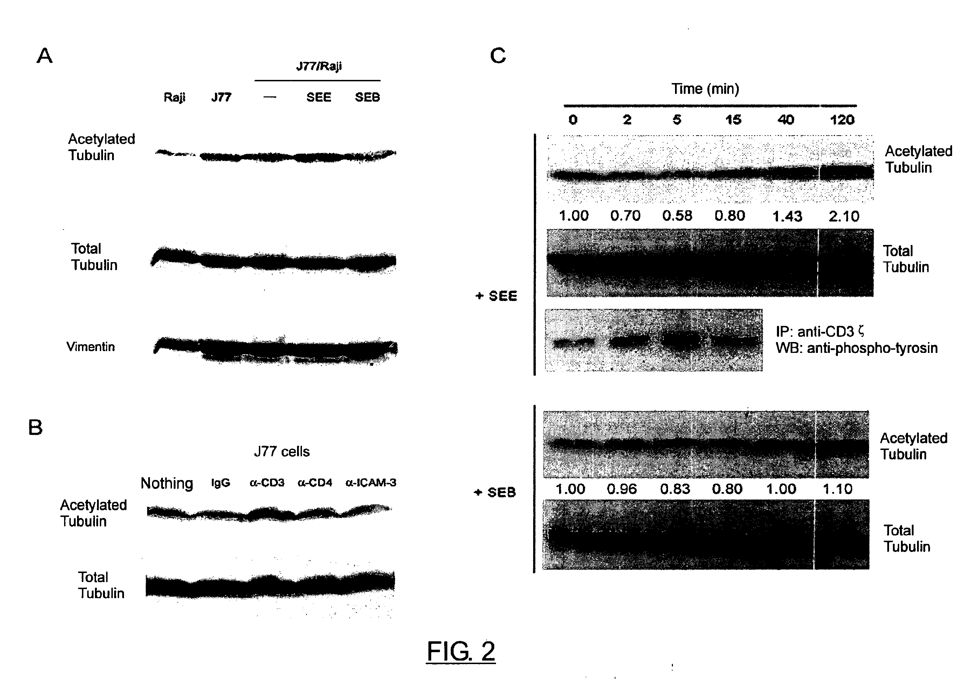

A particular object of the present invention is the compound identification process of the invention in which the determination of tubulin acetylation levels ii) can be performed by means of immunoblot or Western blot techniques with suitable antibodies (see Example 2 and Figure 2). Determination of the effect of compounds on enzymes regulating tubulin, HDAC6 or acetyltransferase acetylation/deacetylation may be carried out in different manners and is widely disclosed in the state of the art: (LeDizet M, Piperno G (1991) Detection of acetylated alpha-tubulin by specific antibodies. Methods Enzymol. 196, 264-274; WO 03/048774, Use of alpha-tubulin acetylation levels as a biomarker for protein deacetylase inhibitors; WO 03/066885, Method for screening for compounds having HDAC6 protein tubulin deacetylase activity inhibitory activity; EP 1243658, Method of measuring deacetylase activity and method of screening inhibitor or promoter of these enzymes; EP 1050581, Method for detecting acetyltransferase and deacetylase activities and method for screening inhibitors or accelerators of these enzymes; PCT application for US patent 20030082668, Method for measuring the activity of deacetylase and method of screening for inhibitors and accelerators of the enzyme). These processes may be applied for identifying regulating, inhibiting and agonist compounds of the HDAC6 protein as a deacetylase protein, and subsequent assessment of the regulating capacity of said compounds on T lymphocyte activation.

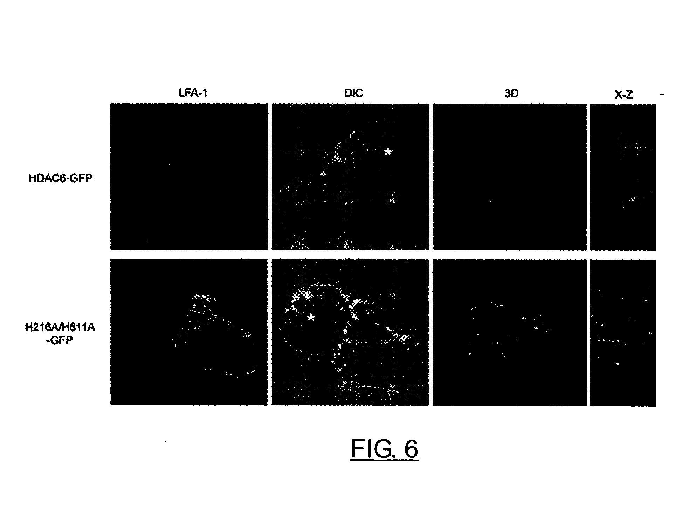

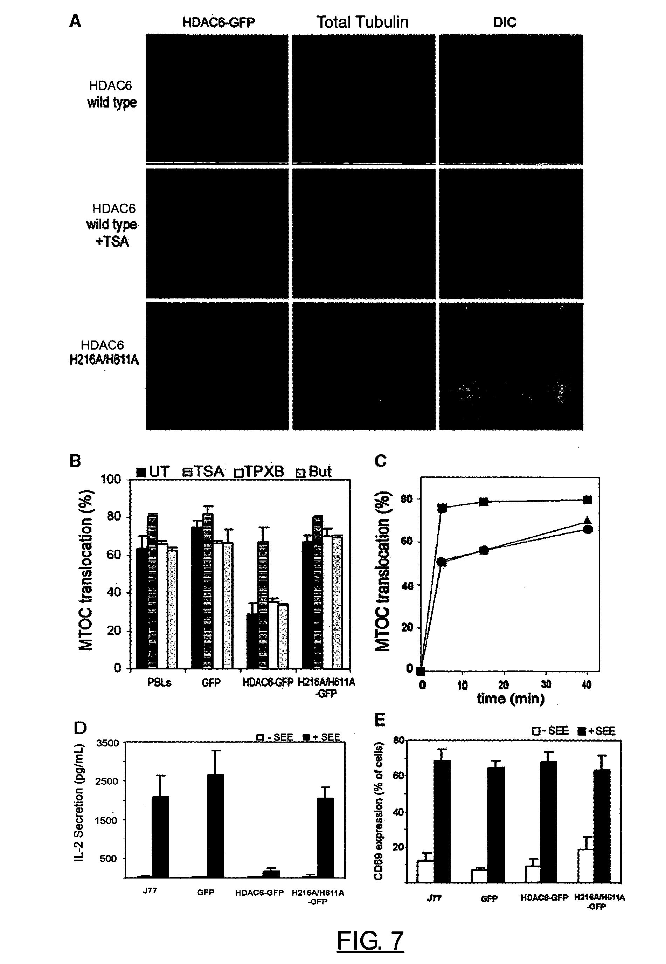

Another particular object of the present invention is the compound identification process of the invention in which the determination of a parameter related to the T lymphocyte activation process ii) can be performed, in an illustrative manner and without limiting the scope of the present invention, by means of a technique belonging to the following group:
a) determining CD3 clustering or concentration in the immunological synapse (c-SMAC) (see Example 4, Figure 5),
b) determining LAF-1 disorganization in the immunological synapse peripheral ring (see Example 4, Figure 6),
c) determining the microtubule organizing centre (MTOC) translocation (see Example 5, Figure 7), and
d) determining IL-2 secretion and CD69 expression (see Example 5, Figure 7).

Preferably human T lymphocytes, as used in the present invention, used in the compound identification process, include CD4+ type cell cultures amongst which are, in an illustrative manner and without limiting the scope of the present invention, the following cells: peripheral blood lymphocytes, T lymphoblasts and Jurkat cells (J77).

On the other hand, the origin of these candidate compounds for the identification process of the invention may be varied, such that they may be of natural origin (for example, of plant, bacterial, viral, animal or eukaryotic microorganism origin) or synthetic.

An additional object of the present invention is a pharmaceutical composition or a medicine for the prophylaxis and treatment of diseases, disorders or pathologies occurring with immune system alterations, preferably with altered T lymphocyte activation, hereinafter pharmaceutical composition of the present invention, comprising a therapeutically effective amount of an HDAC6 protein tubulin deacetylase activity regulating compound or agent optionally together with one or more adjuvants and/or pharmaceutically acceptable vehicles, and which is able to regulate said altered T lymphocyte activation.

Another particular object of the present invention is a pharmaceutical composition for the prophylaxis and treatment of diseases, disorders or pathologies occurring with immune system alterations, preferably with a reduced T lymphocyte activation, comprising a therapeutically effective amount of an HDAC6 protein inhibiting compound optionally together with one or more adjuvants and/or pharmaceutically acceptable vehicles, and which is able to induce T lymphocyte activation.

Another particular object of the present invention is a pharmaceutical composition for the prophylaxis and treatment of diseases, disorders or pathologies occurring with immune system alterations, preferably with an increased T lymphocyte activation, comprising a therapeutically effective amount of an HDAC6 protein activating compound optionally together with one or more adjuvants and/or pharmaceutically acceptable vehicles, and which is able to inhibit T lymphocyte activation.

A particular embodiment of the present invention is a pharmaceutical composition in which the activating compound is a gene construct or an expression vector allowing expression of a peptide or protein mimicking or reproducing human HDAC6 protein tubulin deacetylase biological activity and comprising one of the nucleotide sequences belonging to the following group:
a) a nucleotide sequence (SEQ. ID NO. 1) coding for human HDAC6 protein (SEQ. ID NO. 2),
b) a fragment of the nucleotide sequence of a), and
c) a nucleotide sequence similar to the sequences of a) and b).

The adjuvants and pharmaceutically acceptable vehicles which may be used in said vaccines are the adjuvants and vehicles known by people skilled in the art and normally used in developing therapeutic compositions.

The expression "therapeutically effective amount" in the sense used in this description refers to the amount of the HDAC6 protein tubulin deacetylase activity regulating, inhibiting or agonist compound or agent calculated to produce the desired effect and will generally be determined, amongst others, by the characteristics of the compounds themselves, including age, patient status, the seriousness of the alteration or disorder and administration method and frequency.

In a particular embodiment, said therapeutic composition is prepared in the form of a solid form or an aqueous suspension in a pharmaceutically acceptable diluent. The therapeutic composition provided by this invention can be administered via any suitable administration method, to which end said composition will be formulated in the pharmaceutical form suitable for the chosen administration method. In a particular embodiment, the therapeutic composition provided by this invention is administered parenterally, orally, intraperitoneally, subcutaneously, etc. A review of the different pharmaceutical forms for administering medicines and the excipients required for obtaining them can be found, for example, in the "Tratado de Farmacia Galénica" *"Galenic Pharmacy Treatise",* C. Faulí i Trillo, 1993, Luzán 5, S.A. Ediciones, Madrid.

Another object of the present invention is the use of the pharmaceutical composition of the present invention in a prophylaxis and treatment method for a mammal, preferably a human being, having a disease, disorder or pathology characterized by an undesirable immune response, hereinafter use of the pharmaceutical composition of the present invention, consisting in the administration of said therapeutic composition modifying the tubulin acetylation status and acting as an immunomodulatory composition.

A particular object of the present invention is the use of a pharmaceutical composition of this invention containing an HDAC6 protein inhibiting compound in a prophylaxis and treatment method for a subject having a disease, disorder or pathology requiring, or who may benefit from, an increased immune response, increased by means of administering a compound or agent increasing the tubulin acetylation status in T lymphocytes. Thus, this compound may favor tubulin acetylation by means of inhibiting tubulin deacetylase activity, preferably HDAC6 protein tubulin deacetylase activity, or by means of increasing tubulin acetyltransferase activity. In this case the disease, disorder or pathology may belong, in an illustrative manner and without limiting the scope of the invention, to the following group:
- a disorder or pathology in which the subject would not have a compromised immune response but would benefit from an increased immune response, for example in early stages of a tumor process,
- an immunodeficiency selected from the following group: inherited immunodeficiency disorders, congenital immunodeficiency disorders, radiotherapy-associated immunosuppression syndrome, AIDS and chemotherapy-associated immunosuppression syndrome, and
- preventive situations or treatments with vaccines requiring an increase in the immune response to an antigen (for example: vaccination or response to infections, response to tumors). In this case, the method would enhance the immune response to an antigen comprising administering to the subject an effective amount of an antigen and/or DNA coding for an antigen, preferably included in a vaccine, and an agent increasing the tubulin acetylation status to thus increase the immune response of the subject against the antigen.

Another particular object of the present invention is the use of a pharmaceutical composition of this invention containing an HDAC6 protein activating or agonist compound in a prophylaxis and treatment method for a subject having a disease, disorder or pathology characterized by having an increased immune response regarding the normal response of a healthy subject and who may benefit from a immune response that is reduced by means of the administration of said agonist compound. Thus, this compound may favor tubulin deacetylation by means of increasing tubulin deacetylase activity, preferably HDAC6 protein tubulin deacetylase activity, or by means of reducing tubulin acetyltransferase activity. In this case the disease, disorder or pathology may belong, in an illustrative manner and without limiting the scope of the invention, to the following group:
- acute or chronic inflammatory disorder or an immune disorder, for example an autoimmune disease selected from the group formed by: rheumatoid arthritis, systemic erythematous lupus, sclerodermia, Sjögren's syndrome, autoimmune diabetes, thyroiditis, and other organ-specific immune diseases including psoriasis;
- neurological-type disorder selected from the following group: multiple sclerosis, severe myasthenia, and other neurological immune diseases;
- gastrointestinal-type disorder selected from the following group: Crohn's disease, ulcerous colitis, celiac disease and hepatitis;
- respiratory-type disorder selected from the following group: emphysema, respiratory tract infections;
- cardiovascular-type disorder selected from the following group: atherosclerosis, cardiomyopathy, rheumatic fever, endocarditis, vasculitis and other cardiologic diseases of an immune nature;
- allergic-type disorder or hypersensitivity reaction (types I, II, III and IV) including asthma, rhinitis and other hypersensitivity reactions mediated by the immune system;
- transplant or graft rejection- type disorder, and
- disorder or condition belonging to the following group: acute lung damage, acute respiratory distress syndrome, arthritis (CIA, for example), bronchitis, cystic fibrosis, hepatitis, inflammatory bowel disease, reperfusion damage, nephritis, pancreatitis, arterial occlusion, cerebrovascular accident, systemic erythematous lupus, ultraviolet light-induced damage, vasculitis and sarcoidosis.

The use of the pharmaceutical composition of the present invention may be used in a prophylaxis and treatment method in an isolated manner, or together with other pharmaceutical compounds.

Likewise verified from the results of the present invention is the importance of acetyltransferase proteins, which would act by increasing tubulin acetylation levels observed during the T lymphocyte activation process, and which would allow:
a) developing processes for identifying new regulating, inhibiting and agonist compounds for said T lymphocyte activation (see, for example, EP 1050581, Method for detecting acetyltransferase and deacetylase activities and method for screening inhibitors or accelerators of these enzymes; EP 1243658, Method of measuring deacetylase activity and method of screening inhibitor or promoter of these enzymes),
b) the use of these regulating compounds in developing new therapeutic compositions for the prophylaxis and treatment of diseases with T lymphocyte activation alterations, and
c) the use of said therapeutic compositions in prophylaxis and treatment methods for mammals, preferably humans, having a disease, disorder or pathology characterized by an undesirable immune response, consisting in administrating said therapeutic composition modifying the tubulin acetylation status and acting as an immunomodulatory composition.

### DESCRIPTION OF THE DRAWINGS

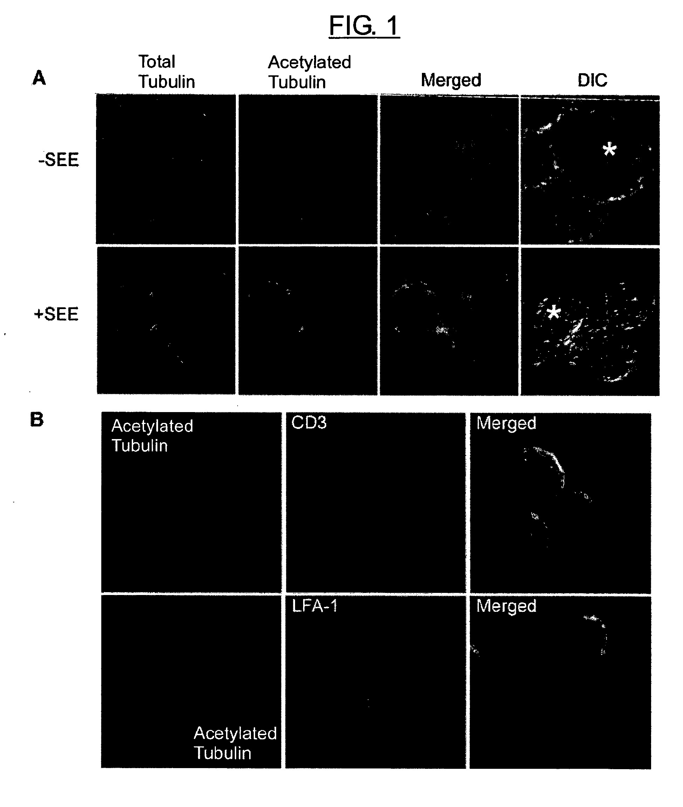

**Figure 1: The acetylated microtubules are concentrated in the contact area between T cells and antigen-presenting cells, surrounding CD3 and LFA-1 clusters.**
   The Raji cells were placed in contact with J77 cells on fibronectin-coated cover slips. The cells were then fixed, permeabilized and stained with the antibodies indicated. **A)** The cell conjugates formed in the absence (upper panel) or presence of SEE at 1 µg/ml (lower panel) were double stained for tubulin (green) and acetylated tubulin (red) and analyzed by confocal microscopy. The superimposed images and the phase contrast images are shown on the right. The asterisks indicate the location of the Raji cells in the cell conjugates. **B)** The J77/Raji cell conjugates in the presence of SEE were stained for acetylated tubulin (red) and CD3 (green, upper panel) or LFA-1 (green, lower panel). The panels on the right show the Raji cell stains (blue), acetylated tubulin (red) and CD3 or LFA-1 stains (green) superimposed on the Nomarski images.
**Figure 2: T cell acetylated tubulin increases after interacting with antigen-presenting cells. A)** The Raji cells (1.5 x 10⁶) were or were not marked with 1µg/ml SEB or SEE and placed in contact with 4.5 x 10⁶ J77 cells for 40 minutes at 37°C. Acetylated tubulin, tubulin and vimentin levels were then detected by immunoblotting in cell lysates under non-reducing conditions, as described in Methods. **B)** The J77 cells (5 x 10⁶) were stimulated with the indicated antibodies for 40 minutes at 37°C. Acetylated tubulin and tubulin levels were then detected by immunoblotting under non-reducing conditions. **C)** Kinetic study of tubulin acetylation and of CD3ζ phosphorylationphosphorylation in conjugates between J77 and Raji cells. The J77 and Raji cells were placed in contact in the presence of SEE or SEB for the times indicated, and acetylated tubulin and tubulin levels were then detected by immunoblotting under non-reducing conditions. The densitometric values are shown between the panels. In order to detect CD3ζ phosphorylationphosphorylation the cell lysates were precipitated with antibody 488, the immunoprecipitates were separated in SDS-PAGE under reducing conditions and the degree of phosphorylationphosphorylation in tyrosine was detected by immunoblotting using antibody 4G10.

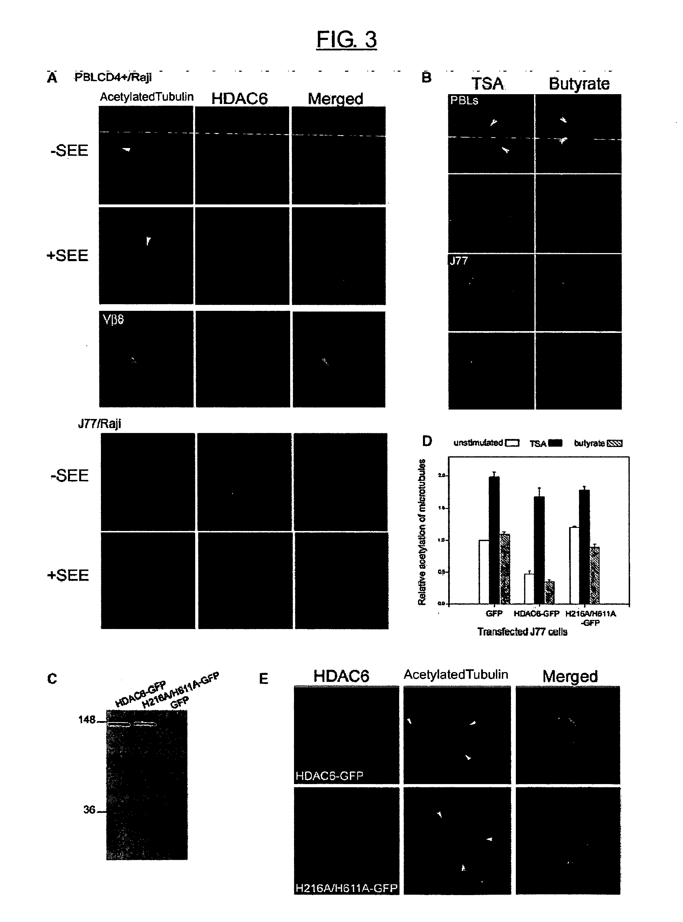

**Figure 3: Characterization of HDAC6 expression in T cells. A)** The HDAC6 enzyme is concentrated in the contact sites between T cells and antigen-presenting cells. Raji cells marked with CMAC (blue) were placed in contact with CD4+ peripheral blood lymphocytes (PBLCD4+) or J77 cells in the presence or absence of SEE. The cells were then fixed, permeabilized and stained for HDAC6 and acetylated tubulin. The arrows indicate the microtubule organizing centers. **B)** The CD4+ peripheral blood lymphocytes and the J77 cells were pre-treated for 30 minutes with 3µM TSA or 5 mM sodium butyrate and were placed in contact with SEE-pulsed Raji cells. Acetylated microtubules were stained in T cell and antigen-presenting cell conjugates. The arrows indicate T cell acetylated microtubules. **C)** Lysates from J77 cells or J77 cells transfected with GFP, HDAC6-GFP or their inactive mutant were processed by immunoblotting with an anti-GFP antibody. The molecular weight markers are shown on the left. **D)** Acetylated tubulin levels in J77 cells transfected with HDAC6. J77 cells were transfected with GFP, HDAC6-GFP or their inactive mutant. After 24 hours the cells were treated or not with 3 µM TSA or 5 mM sodium butyrate and acetylated tubulin levels were determined by flow cytometry as described in Methods. One representative experiment out of three is shown. **E)** Exogenous HDAC6 is concentrated in the contact site between J77 cells and Raji cells. The SEE-marked Raji cells were placed in contact with J77 cells transfected with HDAC6 or an inactive mutant of HDAC6. Cell conjugates were then fixed, permeabilized and stained for acetylated tubulin. The arrowheads indicate the contact area between T cells and antigen-presenting cells.

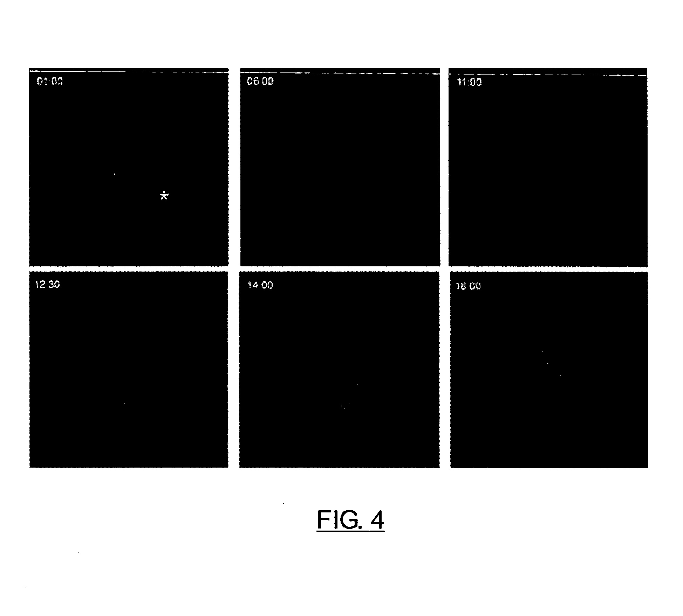

**Figure 4: HDAC6 locating dynamics during antigen-specific interaction between T cells and antigen-presenting cells.** The Raji cells were pulsed with 1 µg/ml SEE and adhered onto fibronectin-coated cover slips. J77 cells transfected with HDAC6 were then added and images were taken at 30 second intervals. The Raji cells were marked with the CMAC blue probe. Each point shows the phase contrast image superimposed on the fluorescent HDAC6-GFP image. The time from the initial contact is indicated in the upper left corner of each image, in minutes. The asterisks indicate the Raji cells.
**Figure 5: HDAC6 overexpression disorganizes the CD3 cluster in the central region of the immunological synapse. A)** The J77 cells were transfected with GFP, HDAC6-GFP or their inactive mutant and were incubated with SEE-marked Raji cells for 30 minutes at 37°C, after which a CD3ζ stain was performed. The XZ projections corresponding to the contact area of each conjugate are shown on the right. Phase contrast images superimposed on CD3ζ staining and HDAC6-GFP are also shown. The asterisks indicate the location of the antigen-presenting cells in the cellular conjugates. **B)** The percentage of CD4+/Vβ8+ peripheral blood lymphocytes (PBLs) and J77 cells transfected with GFP, HDAC6 or the inactive mutant of HDAC6, treated or not with TSA, trapoxin B or sodium butyrate showing CD3 accumulation in the contact site with Raji cells. **C)** The J77 cells pre-treated with TSA ( ), trapoxin B (O) or sodium butyrate (Δ) were placed in contact with Raji cells marked with 1µg/ml SEE and CD3 clustering was analyzed at different times. One representative experiment out of three is shown.
**Figure 6: HDAC6 overexpression disorganizes LFA-1 in the immunological synapse peripheral ring.** SEE-marked Raji cells were incubated for 30 minutes at 37°C with J77 cells transfected with HDAC6-GFP or their inactive mutant. The cells were then stained for LFA-1 and analyzed by confocal microscopy. Representative confocal sections and three-dimensional reconstructions of T cell and antigen-presenting cell conjugates in which the J77 cells overexpressed HDAC6-GFP (upper-panel) or its inactive mutant (lower panel) are shown. Twenty cell conjugates corresponding to three independent experiments for each transfectant were analyzed. LFA-1 was detected in the peripheral ring of 9 (GFP), 2 (HDAC6-GFP), and 11 (H216A/H611A-GFP) cells. The XZ projections corresponding to the binding area of each transfectant with the antigen-presenting cell are shown on the right. The phase contrast image is also shown superimposed on the LFA-1 stain in cells transfected with HDAC6-GFP or its inactive mutant. The asterisks indicate the antigen-presenting cells.
**Figure 7: Functional role of HDAC6 in microtubule organizing centre translocation and T cell activation. A)** The J77 cells transfected with HDAC6 or the inactive mutant of HDAC6 were incubated with SEE-pulsated Raji cells at 37°C for 30 minutes. The cells were then fixed, permeabilized and stained for tubulin. Representative images of cell conjugates expressing HDAC6-GFP in the absence and presence of 3 µM TSA (upper and middle panels, respectively) and cells expressing the inactive mutant of HDAC6 are shown. The phase contrast images are also shown superimposed on HDAC6 and tubulin stains. **B)** The SEE-pulsed Raji cells were placed in contact with CD4+/Vβ8+ peripheral blood lymphocytes in the presence or absence of TSA, trapoxin B or sodium butyrate and the reorientation of the microtubule organizing centre was recorded by direct visualization of T cells with the microtubule organizing centre in close proximity to the plasmatic membrane between the nucleus and the contact site with the Raji cell. The data corresponds to the arithmetic mean ± the standard error of four independent experiments. At least 200 conjugates were counted per experiment. **C)** J77 cells were pre-treated with TSA ( ), trapoxin B (O) or sodium butyrate (Δ) and placed in contact with SEE-pulsed Raji cells. Microtubule organizing centre translocation was determined at different times. One representative experiment out of three is shown. **D)** IL-2 concentration was determined in the supernatant of conjugates between Raji cells and J77 cells stably transfected with GFP, HDAC6-GFP or their inactive mutant in the presence (+SEE) or absence (-SEE) of 0.5 µg/ml of SEE. The results show the arithmetic mean ± the standard deviation of IL-2 production in pg/ml corresponding to three independent experiments. **E)** As in D, the number of cells positive for CD69 amongst the J77 Vβ8 positive cells in conjugates was determined. The results are expressed as the arithmetic mean ± the standard deviation of the percentage of CD69+ cells in the Vβ8+ subpopulation for three independent experiments.

### PATENT EXAMPLES

The following examples allow illustrating the invention and must not be considered as limiting to the scope thereof.

### Example 1: Microtubules are acetylated in the contact site of T cells with antigen-presenting cells.

The cell conjugates between T cells and antigen-presenting cells in the presence of the *Staphylococcus* enterotoxic superantigen showed a concentration of acetylated microtubules in the contact area with the antigen-presenting cell (Figure 1 A). The amount of acetylated microtubules was larger in superantigen-specific conjugates than in conjugates between cells of the same type or cell conjugates established in the absence of superantigen E (Figure 1A). Furthermore, acetylated T cell microtubules were found surrounding the typical central and peripheral immunological synapse markers such as CD3 and adhesion molecule LFA-1, respectively (Figure 1 B).

### Example 2: Microtubule acetylation increases with the presence of superantigen in T cell and antigen-presenting cell conjugates.

Immunoblot analysis showed that tubulin acetylation levels increased significantly in conjugates between T cells and antigen-presenting cells in the presence of superantigen E but not in the presence of superantigen B (Figure 2A).

In order to confirm that the increase in microtubule acetylation took place in T cells, the effect of CD3 crossover by means of monoclonal antibodies directed at this molecule was examined. Tubulin acetylation basal levels in T cells increased with treatment with said antibodies (Figure 2B). In contrast, tubulin acetylation levels were not modified in the presence of anti-CD4, anti-ICAM-3 antibodies or other irrelevant immunoglobulins (Figure 2B). Tubulin acetylation kinetics were determined during immunological synapse formation between T cells and antigen-presenting cells in the presence of superantigen E. A transitory deacetylation of tubulin was observed at short times (2-3 minutes), recovering basal levels after the first 15 minutes. From this moment onwards tubulin acetylation increased progressively, reaching a maximum level after two hours of stimulation (Figure 2C). In contrast, acetylation levels did not vary in the presence of superantigen B (Figure 2C). This data clearly showed that two phases could be distinguished during superantigen-mediated tubulin acetylation. Tubulin deacetylation kinetics clearly coincided with tyrosine phosphorylation kinetics in the CD3 ζ chain (Rozdzial, M.M., Malissen, B., and Finkel, T.H. (1995). Tyrosine-phosphorylated T cell receptor zeta chain associates with the actin cytoskeleton upon activation of mature T lymphocytes. Immunity 3, 623-633) induced by superantigen E binding to the T cell receptor (Figure 2C).

### Example 3: Antigen-induced microtubule acetylation in the T cell is regulated by HDAC6

It was observed by means of double immunofluorescence assays that HDAC6 is located in the cytoplasm of Jurkat cells or peripheral blood CD4+ lymphocytes conjugated with superantigen E-marked Raji cells. HDAC6 was partially co-located with both cell type acetylated microtubules, concentrated in the contact site with the antigen-presenting cell (Figure 3A). In contrast, HDAC6 was located in a disperse manner in conjugates between T cells and antigen-presenting cells in the absence of superantigen E (Figure 3A). In order to determine whether tubulin deacetylating activity occurred in the contact site between the T cell and the antigen-presenting cell, tubulin acetylation was detected in the presence of TSA, an HDAC6 tubulin deacetylase activity specific inhibitor, sodium butyrate and trapoxin B, two potent deacetylase histone inhibitors incapable of inactivating HDAC6 (Hubbert, C., Guardiola, A., Shao, R., Kawaguchi, Y., Ito, A., Nixon, A., Yoshida, M., Wang, X., and Yao, T. (2002). HDAC6 is a microtubule-associated deacetylase. Nature 417, 455-458; Furumai, R., Komatsu, Y., Nishino, N., Khochbin, S., and Yoshida, M. (2001). Potent histone deacetylase inhibitors built from trichostatin A and cyclic tetrapeptide antibiotics including trapoxin. Proc. Natl. Acad. Sci. USA 98, 87-92). CD4+ T lymphocytes and J77 cells pretreated with TSA showed strong acetylated tubulin staining in the contact site with antigen-presenting cells. However, staining was weak both in untreated cells and in cells treated with butyrate or with trapoxin B (Figure 3B and data not shown), indicating that HDAC6 was active in that cell location.

In order to analyze the role of HDAC6 in superantigen-specific cell conjugates in more detail, HDAC6 fusion proteins bound to the green fluorescent protein (GFP) at the carboxyl end (HDAC6-GFP) were generated and another GFP construct of an inactive HDAC6 protein mutant was also generated, the histidines in positions 216 and 611 of which were changed for alanines (H216A/H611A-GFP) (SEQ ID NO. 2 protein with histidine modifications in positions 216 and 611) (Figure 3C). These proteins were temporarily expressed in the Jurkat cell clone J77, which showed a moderate expression of endogenous HDAC6. Tubulin acetylation levels in the presence of TSA or sodium butyrate were determined by flow cytometry. Tubulin acetylation was found to increase in cells due to TSA action (Figure 3D). However, cells expressing HDAC6-GFP had lower tubulin levels than cells transfected with the inactive mutant or GFP (Figure 3D). On the other hand, when the J77 cells expressing HDAC6-GFP were placed in contact with Raji cells in the presence of superantigen E, HDAC6 was excluded from the nucleus and concentrated in the contact area between the T cell and the antigen-presenting cell. This contact area showed a lower amount of acetylated microtubules in cells expressing HDAC6-GFP than in non-transfected cells (Figure 3E, upper panel arrowheads). In the case of cells expressing the HDAC6 inactive mutant, tubulin acetylation did not decrease in the contact site despite observing a similar location for HDAC6-GFP or its tubulin deacetylase activity-deficient form (Figure 3E, lower panel arrowheads). On the whole, these results show that HDAC6 polarizes towards the contact area with the antigen-presenting cell where its enzymatic activity takes place.

### Example 4: HDAC6 is redistributed from the central area towards the peripheral area of the intercellular contact and regulates organization of the immunological synapse during formation of T cell and antigen-presenting cell conjugates.

Confocal fluorescence videomicroscopy of T cells transfected with HDAC6-GFP and placed in contact with antigen-presenting cells in the presence of superantigen E was carried out in order to study the location of HDAC6 in the contact area of the T cell with the antigen-presenting cell. At the very beginning of the contact, HDAC6-GFP was located in the membrane regions oriented towards the antigen-presenting cell, concentrated in the central area of the cellular contact (Figure 4). After six minutes of contact, HDAC6-GFP redistributed towards the periphery (Figure 4). Finally, after 11 minutes, HDAC6-GFP was excluded from the central area, concentrated in a peripheral ring similar to the one characteristic of the immunological synapse (Figure 4). In the case of analyzing the HDAC6 inactive mutant a similar location was observed, indicating that positioning of the enzyme is independent from its enzymatic activity.

Immunological synapse maturing is correlated to CD3 clustering in a small central area and LFA-1 in a peripheral ring (Monks, C.R., Friberg, B.A., Kupfer, H., Sciaky, N., and Kupfer, A. (1998). Three-dimensional segregation of supramolecular activation clusters in T cells. Nature 395, 82-86; Grakoui, A., Bromley, S.K., Sumen, C., Davis, M.M., Shaw, A.S., Allen, P.M., and Dustin M.L., (1999). The immunological synapse: a molecular machine controlling T cell activation 285, 221-227). In order to determine if HDAC6 plays an important role in immunological synapse organization, the location of CD3 and LFA-1 was determined in the T cells transfected with HDAC6-GFP synapse and forming conjugates with antigen-presenting cells in the presence of superantigen E. The presence of HDAC6-GFP, but not that of its inactive mutant, prevented CD3 clustering in the central region of the immunological synapse (Figure 5A). This effect was reverted by treatment with TSA but not with sodium butyrate or trapoxin B (Figures 5A and B). Furthermore, cells pretreated with TSA always showed a slight increase in CD3 clustering (Figure 5B). The percentage of mature synapses in J77 cells transfected with HDAC6 was two times less than in cells transfected with the acetylation deficient mutant or than lymphocytes treated with TSA and neither trapoxin B nor sodium butyrate modified it in a significant manner (Figure 5B). Finally, kinetic studies of conjugates between T cells and antigen-presenting cells in the presence of superantigen E showed that pretreating the T cells with TSA accelerated CD3 clustering in the central region of the immunological synapse (Figure 5C).

On the other hand, the exogenous expression of HDAC6-GFP prevented normal location of LFA-1 in the peripheral ring of the immunological synapse (Figure 6, upper panel), despite LFA-1 being concentrated in the cell conjugate contact area (data not shown). LFA-1 was well organized in the peripheral ring of the immunological synapse of cells treated with TSA or transfected with the deacetylase activity-deficient HDAC6 mutant (Figure 6, lower panel and data not shown). This data suggests that HDAC6 regulates CD3 and LFA-1 organization in the immunological synapse by means of deacetylating the microtubules surrounding it.

### Example 5: The role of HDAC6 in microtubule organizing centre translocation and in delayed T cell activation T.

After recognizing the antigen, the T cell redirects the Microtubule Organizing Centre (MTOC) towards the antigen-presenting cell as a result of the organization and maturation of the immunological synapse (Podack, E.R., and Kupfer, A. (1991). T-cell effector functions: mechanisms for delivery of cytotoxicity and help. Annu. Rev. Cell Biol. 7, 479-504; Sancho, D., Montoya, M.C., Monjas, A., Gordón-Alonso, M., Katagiri, T., Gil, D., Tejedor, R., Alarcón, B., and Sánchez-Madrid, F. (2002). TCR engagement induces proline-rich tyrosine kinase-2 (Pyk2) translocation to the T cell-APC interface independently of Pyk2 activity and in an immunoreceptor tyrosine-based activation motif-mediated fashion. J. Immunol. 169, 292-300). Therefore, it was investigated whether HDAC6 played any role in MTOC translocation. Cells transfected with HDAC6-GFP did not redirect their MTOC towards the antigen-presenting cells (Figures 7A and B), whereas treatment with TSA, but not with sodium butyrate or trapoxin B, restored normal translocation (Figures 7A and B). On the other hand, the deacetylase activity-deficient HDAC6 mutant did not modify MTOC redirection (Figures 7A and B). Quantitative analysis of these experiments showed that the percentage of cells with a translocated MTOC in cells transfected with HDAC6 was half that of cells treated with TSA or transfected with the deacetylase activity-deficient HDAC6 mutant (Figure 7B). In these experiments, treatment with butyrate or trapoxin B did not affect MTOC translocation (Figure 7B). As described for CD3 clustering, treatment with TSA increases MTOC translocation in peripheral blood CD4+ lymphocytes and accelerates it in cell conjugates between J77 and Raji cells (Figures 7B and C). The effect of HDAC6 overexpression on other activation events was also studied, specifically in IL-2 production and in activation marker CD69 expression (Figures 7D and E). After 6 hours from conjugate formation in the presence of superantigen E, IL-2 production drastically decreased in cells transfected with HDAC6-GFP, whereas this functional response was not affected in cells transfected with the deacetylase activity-deficient HDAC6 mutant (Figure 7D). In contrast, CD69 expression was equally stimulated in all studied cells without being affected by any treatment (Figure 7E).

This data demonstrates that HDAC6 plays a dual role in MTOC redirection, regulating the spatial organization of the immunological synapse membrane receptors and controlling the reorganization of the microtubule network taking place during the formation of conjugates between T cells and antigen-presenting cells.

The Materials and Methods used for carrying out the examples of the present invention are described in detail below.

### MATERIALS AND METHODS

### Antibodies, Cells and Reagents

Anti-CD3 antibody T3b, anti-LFA-1 antibody TP1/40, anti-ICAM-3 antibody TP1/24 and anti-CD4 antibody HP2/6 have been previously disclosed (Campanero, M.R., del Pozo, M.A., Arroyo, A.G., Sánchez-Mateos, P., Hernandez, T., Craig, A., Pulido, R., and Sánchez-Madrid, F. (1993). ICAM-3 interacts with LFA-1 and regulates the LFA-1/ICAM-1 cell adhesion pathway. J. Cell Biol. 123: 1007-1016). Polyclonal antibody 488 was generously granted by Dr. B. Alarcón (Centro de Biología Molecular, Madrid) and the anti-HDAC6 affinity purified rabbit polyclonal antibody was acquired through MBL (Watertown, MO). Anti-α-tubulin antibody B-5-1-2, anti-acetylated α-tubulin antibody 6-11 B-1 and anti-vimentin as well as the rabbit polyclonal antibody against mice IgGs were acquired through Sigma Chemical Co. (St. Louis, MO). Anti-GFP antibody 290 came from Abcam (Cambridge, UK) and the FITC-marked anti-CD3 and anti-α-tubulin antibodies were acquired from Becton Dickinson (San José, CA) and Sigma, respectively.

Peripheral blood human CD4+ lymphocytes were purified by negative selection using the *Human T Cell CD4 Subset Column Kit* (R&D systems, MN) following manufacturer instructions. The J77 human T cell line and the Raji lymphoblastoid B cell line were cultured in RPMI 1640 medium (Gibco BRL, Gaithersburg, MD) supplemented with 10% FCS. SEE was acquired from Toxin Technology (Saratosa, FL). SEB, recombinant human fibronectin and poly-L-lysine (PLL) were obtained from Sigma. Trichostatin A (TSA) and sodium butyrate were obtained from Calbiochem (San Diego, CA). Trapoxin B was generously granted by Dr. Yoshida (Chemical Genetics Laboratory RIKEN, Saitama, Japan). The 80 kD fibronectin fragment (FN80) was generously granted by Dr. A. García-Pardo (CIB, Madrid). The chloromethyl aminocoumarin (CMAC) derivative fluorescent probe came from Molecular Probes (Eugene, OR).

### Immunofluorescence, Immunoprecipitation and Immunoblot Analysis.

The Raji cells were marked with the CMAC fluorescent probe, incubated for 15 minutes in the presence or absence of 1µg/ml of SEE or SEB, and were then placed in contact with the same number of J77 cells transfected with HDAC6-GFP (2x20⁵ cells). Then, the cells were centrifuged at a low speed and incubated for 15 minutes at 37°C. The cell conjugates were gently resuspended, plated on PLL-coated cover slips (80µl/slip) and placed for 15 more minutes in the cell incubator at 37°C. The cells were fixed and permeabilized for 5 minutes in PBS containing 2% formaldehyde-0.5% Triton X-100, and stained for α-tubulin, acetylated α-tubulin, CD3, LFA-1 or HDAC6 using the suitable primary antibodies and secondary antibodies marked with Alexa 488 or rhodamine red X (Molecular Probes). The cell conjugates were observed using a Leica DMR microscope (Leica, Heidelberg, Germany) with 100X immersion lenses and a Leica TCS-SP confocal microscope. The confocal sections were spaced 0.2 µm on the z axis. A quantitative analysis of the conjugates containing J77 cells with CD3 clustered in the central region of the intercellular contact was also carried out in more than 200 conjugates corresponding to three independent experiments. Immunoprecipitation was carried out in a similar manner to that previously described (Sancho, D., Montoya, M.C., Monjas, A., Gordón-Alonso, M., Katagiri, T., Gil, D., Tejedor, R., Alarcón, B., and Sánchez-Madrid, F. (2002). TCR engagement induces proline-rich tyrosine kinase-2 (Pyk2) translocation to the T cell-APC interface independently of Pyk2 activity and in an immunoreceptor tyrosine-based activation motif-mediated fashion. J. Immunol. 169, 292-300). For the immunoblot experiments, 4.5x10⁶ J77 cells were placed in contact with 1.5x10⁶ Raji cells in the presence or absence of SEE or SEB in a 500 µl volume for an identical time under continuous rotation. The cells were then washed once in PBS, resuspended in 60 µl of MES buffer (10mM MES at pH 7.4, 150 mM NaCl, 5 mM EGTA, 5 mM MgCl₂) in the presence of a protease inhibitor cocktail (Roche, Manheim, Germany), they were sonicated twice at 4°C for 30 seconds and boiled for 5 minutes at 100°C in Laemmli buffer containing 5% β-mercaptoethanol or lodoacetamide. The cell lysates were then centrifuged at 10,000x g for 20 minutes and the supernatants were analyzed by SDS-PAGE and immunoblot using anti-α-tubulin and anti-acetylated α-tubulin antibodies as previously described (Piperno, G., and Fuller, M.T. (1985). Monoclonal antibodies specific for an acetylated form of a-tubulin recognize the antigen in cilia and flagella from a variety of organisms. J. Cell Biol. 101, 2085-2094).

### Recombinant DNA Constructs and Cell Transfection.

An HDAC6 construct lacking the ending codon was generated by PCR for HDAC6-GFP expression, using HDAC6 cDNA (Grozinger et al., 1998) as a mould (SEQ ID NO. 1) and oligonucleotides 5' [CGGCGTCGAC GATGACCTCAACCGGC] (SEQ ID NO. 3) and 3' [CGGCCACCGGTGGGTGTGGGTGGGGCATATCCTCCCC] (SEQ ID NO. 4). Once digested with Sall and Agel, the product resulting from the amplification was subcloned into plasmid pEGFP-N1 (Clontech, Palo Alto, CA). The construct coding for the inactive double mutant of HDAC6 H216A/H611A was generated using the QuickchangeTM directed mutagenesis kit (Stratagene, La Jolla, CA) following manufacturer instructions. Substitution of the CAC codon for GCC was verified by sequencing the nucleotide sequence coding for the HDAC6 domains. The J77 cells were temporarily transfected by electroporation, basically as has been previously described (Lowin-Kropf, B., Shapiro, V.S., and Weiss, A. (1998). Cytoskeletal polarization of T cells is regulated by an immunoreceptor tyrosine-based activation motif-dependent mechanism. J. Cell Biol. 140: 861-871). Viable cells 24 hours after being transfected were isolated by centrifugation in a Ficoll gradient and placed in contact with Raji cells to carry out functional studies.

### Measuring Deacetylase Activity

In order to determine tubulin acetylation levels in J77 cells transfected with HDAC6, these cells (2x10⁶ cells/ml) were fixed and permeabilized for 5 minutes in PBS containing 1% paraformaldehyde and 0.03% saponin. The cells were then stained using anti-acetylated-α-tubulin antibody 6-11 B-1 and a goat anti-mice antibody marked with APC (PharMingen, Becton Dickinson). Data acquisition and subsequent analysis were carried out in a FACsCalibur cytometer using Cellquest software (Becton Dickinson).

### Real-time Confocal Microscopy

The cover slips coated with cellular conjugates were mounted on Attofluor open cameras (Molecular Probes, Eugene, OR) and placed on the microscope stage. The cells were kept at 37°C in a 5% CO₂ atmosphere using an incubation system (La-con (GBr) Pe-con (GMBH)). The confocal images were acquired using a Leica TCS-SP confocal microscope (Leica Microsystems, Heidelberg, Germany). Series of fluorescence and differential interference contrast images of J77 cells transfected with HDAC6 and in contact from the beginning with SEE-pulsed Raji cells were obtained every 30 seconds. Six confocal sections were required to capture the entire fluorescence signal. The orthogonal sections of the green fluorescence signal corresponding to HDAC6-GFP were superimposed on the corresponding phase contrast images.

### Microtubule Organizing Centre Translocation Assays

A 1:1 ratio of Raji cells and T cells transfected with HDAC6 were placed in contact for 15 minutes at 37°C in RPMI medium. The cell conjugates were then gently resuspended and plated on PLL-coated cover slips for 15 more minutes as previously described (Sancho et al., 2002). The cell conjugates were then fixed, permeabilized and stained for α-tubulin for the purpose of observation by immunofluorescence microscopy. The MTOC was considered to be redirected when it was in close proximity with the T cell plasma membrane, between the cell nucleus and the contact area with the antigen-presenting cell. At least 200 cell conjugates were counted in each experiment.

### Antigen-Dependent Activation Analysis

The J77 cell line and the stable transfectants derived from it which expressed GFP, HDAC6-GFP and H216A/H611A-GFP were placed in contact with Raji cells at 37°C for 6 hours in the presence or absence of SEE (0.5 µg/ml). IL-2 production in the supernatants and CD69 expression in T cells was then determined. In order to distinguish between Raji B cells from Vβ8 positive J77 cells, the cells were doubly stained using an anti CD69 PE antibody (Leu23, Becton Dickinson) and an anti-Vβ8 biotinyl antibody (Becton Dickinson-PharMingen) plus Estreptavidin-APC. The IL-2 Elipair kit (Diaclone, Besançon, France) was used following manufacturer instructions to determine IL-2 concentration.

## Claims

1. The use of an HDAC6 protein tubulin deacetylase activity regulating, inhibiting or activating compound or agent in developing pharmaceutical compositions for the prophylaxis and treatment of diseases, disorders or pathologies in mammals, preferably humans, occurring with immune system alterations, preferably with altered T lymphocyte activation.

2. The use of a compound according to claim 1, **characterized in that** the regulating compound is an HDAC6 protein tubulin deacetylase activity inhibitor or antagonist inducing T lymphocyte activation.

3. The use of a compound according to claim 2, **characterized in that** the HDAC6 protein activity inhibiting compound is a molecule which, when binding or interacting with said proteins or with functional fragments thereof, reduces or eliminates the intensity or duration of HDAC6 protein tubulin deacetylase activity, or prevents or reduces the expression of the coding gene for said protein.

4. The use of a compound according to claim 3, **characterized in that** the inhibiting compound is a nucleic acid preventing or reducing the expression of the gene coding for the human HDAC6 protein including a nucleotide sequence selected from:
a) an antisense nucleotide sequence specific to the HDAC6 protein gene or mRNA sequence,
b) a ribozyme specific to HDAC6 protein mRNA,
c) an aptamer specific to HDAC6 protein mRNA, and
d) an interference RNA (RNAi) specific to HDAC6 protein mRNA.

5. The use of a compound according to claim 4, **characterized in that** the nucleic acid is a genetic construct or an expression vector allowing expression of sequences a), b), c) and d) inside the cells.

6. The use of a compound according to claim 3, **characterized in that** the inhibiting compound is a chemical compound belonging to the following group: tubacin and hydroxamic acid derivatives.

7. The use of a compound according to claim 6, **characterized in that** the hydroxamic acid derivative is trichostatin (TSA).

8. The use of a compound according to claim 1 **characterized in that** the regulating compound is an HDAC6 protein tubulin deacetylase activity activator, inductor or agonist, inhibiting T lymphocyte activation.

9. The use of a compound according to claim 8, **characterized in that** the HDAC6 protein tubulin deacetylase activity activating compound is a molecule which, when binding or interacting with said protein or with functional fragments thereof, increases the intensity or extends the duration of the HDAC6 protein tubulin deacetylase biological activity, or allows increasing the expression of the gene coding for said protein.

10. The use of a compound according to claim 9, **characterized in that** the activating compound is a xanthine.

11. The use of a compound according to claim 9 **characterized in that** the activating compound is a genetic construct or expression vector allowing the expression of a peptide or protein inside human cells, preferably in T lymphocytes, mimicking or reproducing human HDAC6 protein tubulin deacetylase biological activity, and **in that** it comprises a nucleotide sequence belonging to the following group:
a) a nucleotide sequence (SEQ ID NO. 1) coding for human HDAC6 protein (SEQ ID NO. 2),
b) a fragment of the nucleotide sequence of a), and
c) a nucleotide sequence similar to the sequences of a) and b).

12. A process of identifying and evaluating the activity of HDAC6 protein regulating compounds on T lymphocyte activation, **characterized in that** it comprises the following steps:
i) the contact of preferably human T lymphocytes with the potential compound object of this process and incubation in suitable conditions,
ii) determination of a parameter indicating tubulin acetylation levels in the T lymphocytes of i) or of any other parameter related to the T lymphocyte activation process in which the HDAC6 protein participates, and
iii) identification of an HDAC6 protein activity inhibiting or agonist compound when an increase in tubulin acetylation and/or a parameter indicating T lymphocyte activation is observed, or when a reduction in tubulin acetylation and/or a parameter indicating T lymphocyte inactivation is observed, respectively.

13. A process of identifying potential inhibitors according to claim 12, **characterized in that** the T lymphocytes of point i) may be stimulated or prepared by means of processes, *inter alia,* belonging to the following group: stimulation with antigen-presenting cells (Raji cells, for example) and SEE and transformation of T lymphocyte cell cultures with a genetic construct allowing expression of the HDAC6 gene and allowing a better evaluation of the effect of said potential inhibiting compounds.

14. A compound identification process according to claim 12, **characterized in that** determination of tubulin acetylation levels of ii) is carried out by means of immunoblotting or Western blot techniques with suitable antibodies.

15. A compound identification process according to claim 12, **characterized in that** determination of a parameter related with the activation process of the T lymphocytes of ii) is performed, amongst other possible techniques, by means of a technique belonging to the following group:
i) determining CD3 clustering or concentration in the immunological synapse (c-SMAC),
ii) determining LAF-1 disorganization in the immunological synapse peripheral ring,
iii) determining the microtubule organizing centre (MTOC) translocation, and
iv) determining IL-2 secretion and CD69 expression.

16. A pharmaceutical composition for the prophylaxis and treatment of diseases, disorders or pathologies occurring with immune system alterations, preferably with altered T lymphocyte activation, **characterized in that** it comprises a therapeutically effective amount of an HDAC6 protein tubulin deacetylase activity regulating compound or agent, optionally together with one or more adjuvants and/or pharmaceutically acceptable vehicles, and which is capable of regulating said altered T lymphocyte activation.

17. A pharmaceutical composition according to claim 16 for the prophylaxis and treatment of a pathology occurring with immune system alterations, preferably with reduced T lymphocyte activation, **characterized in that** it comprises a therapeutically effective amount of an HDAC6 protein tubulin deacetylase activity inhibiting compound, optionally together with one or more adjuvants and/or pharmaceutically acceptable vehicles, and which is capable of inducing T lymphocyte activation.

18. A pharmaceutical composition according to claim 16 for the prophylaxis and treatment of a pathology occurring with immune system alterations, preferably with increased T lymphocyte activation, **characterized in that** it comprises a therapeutically effective amount of an HDAC6 protein tubulin deacetylase activity activating compound, optionally together with one or more adjuvants and/or pharmaceutically acceptable vehicles, and which is capable of inhibiting T lymphocyte activation.

19. A pharmaceutical composition according to claim 18, **characterized in that** the activating compound is a genetic construct or an expression vector allowing the expression of a peptide or protein mimicking or reproducing human HDAC6 protein tubulin deacetylase biological activity and comprising one of the nucleotide sequences belonging to the following group:
a) a nucleotide sequence (SEQ. ID NO. 1) coding for human HDAC6 protein,
b) a fragment of the nucleotide sequence of a), and
c) a nucleotide sequence similar to the sequences of a) and b).

20. The use of a pharmaceutical composition according to claims 16 to 19 in prophylaxis and treatment method in a mammal, preferably a human being, having a disease, disorder or pathology **characterized by** an undesirable immune response, consisting in the administration of said therapeutic composition which modifying the tubulin acetylation status and acting as an immunomodulatory composition.

21. The use of the pharmaceutical composition according to claim 20, **characterized in that** the pharmaceutical composition contains an HDAC6 protein tubulin deacetylase activity inhibiting compound, and **in that** the method is a prophylaxis and treatment method in a subject having a disease, disorder or pathology requiring, or who may benefit from, an immune response increased by means of administering a compound or agent increasing the tubulin acetylation status in T lymphocytes, and **in that** the disease belongs, amongst others, to the following group:
- a disorder or pathology in which the subject would not have a compromised immune response but would benefit from an increased immune response, for example in early stages of a tumor process,
- an immunodeficiency selected from the following group: inherited immunodeficiency disorders, congenital immunodeficiency disorders, radiotherapy-associated immunosuppression syndrome, AIDS and chemotherapy-associated immunosuppression syndrome, and
- preventive situations or treatments with vaccines requiring an increase in the immune response to an antigen (for example: vaccination or response to infections, response to tumors) in which the method would enhance the immune response to an antigen comprising administering to the subject an effective amount of an antigen and/or DNA coding for an antigen, preferably included in a vaccine, and an agent increasing the tubulin acetylation status to thus increase the immune response of the subject against the antigen.

22. The use of the pharmaceutical composition according to claim 20, **characterized in that** the pharmaceutical composition contains an HDAC6 protein tubulin deacetylase activity activating or agonist compound, and **in that** the method is a prophylaxis and treatment method for a patient having a disease, disorder or pathology having an increased immune response with respect to a healthy subject and who may benefit from a immune response reduced by means of the administration of said agonist compound, and **in that** the disease belongs, amongst others, to the following group:
- acute or chronic inflammatory disorder or an immune disorder, for example an autoimmune disease selected from the group consisting of: rheumatoid arthritis, systemic erythematous lupus, sclerodermia, Sjögren's syndrome, autoimmune diabetes, thyroiditis, and other organ-specific immune diseases including psoriasis;
- neurological-type disorder selected from the following group: multiple sclerosis, severe myasthenia, and other neurological immune diseases;
- gastrointestinal-type disorder selected from the following group: Crohn's disease, ulcerous colitis, celiac disease and hepatitis;
- respiratory-type disorder selected from the following group: emphysema, respiratory tract infections;
- cardiovascular-type disorder selected from the following group: atherosclerosis, cardiomyopathy, rheumatic fever, endocarditis, vasculitis and other cardiologic diseases of an immune nature;
- allergic-type disorder or hypersensitivity reaction (types I, II, III and IV) including asthma, rhinitis and other hypersensitivity reactions mediated by the immune system;
- transplant or graft rejection- type disorder, and
- disorder or condition belonging to the following group: acute lung damage, acute respiratory distress syndrome, arthritis (CIA, for example), bronchitis, cystic fibrosis, hepatitis, inflammatory bowel disease, reperfusion damage, nephritis, pancreatitis, arterial occlusion, cerebrovascular accident, systemic erythematous lupus, ultraviolet light-induced damage, vasculitis and sarcoidosis.
